(19)

Europäisches
Patentamt
European
Patent Office
Office européen
des brevets

(11) **EP 4 269 435 A1**

(12) **EUROPEAN PATENT APPLICATION**
published in accordance with Art. 153(4) EPC

(43) Date of publication:
**01.11.2023 Bulletin 2023/44**

(21) Application number: **21909462.0**

(22) Date of filing: **22.12.2021**

(51) International Patent Classification (IPC):
*C07K 16/28* (2006.01) *A61K 39/00* (2006.01)

(52) Cooperative Patent Classification (CPC):
**A61K 39/00; C07K 16/28**

(86) International application number:
**PCT/CN2021/140449**

(87) International publication number:
**WO 2022/135467 (30.06.2022 Gazette 2022/26)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **23.12.2020 CN 202011544445**

(71) Applicant: **Innovent Biologics (Singapore) Pte.
Ltd.
Singapore 189767 (SG)**

(72) Inventors:
• **LI, Li
Suzhou, Jiangsu 215123 (CN)**

• **FU, Fenggen
Suzhou, Jiangsu 215123 (CN)**
• **NI, Haiqing
Suzhou, Jiangsu 215123 (CN)**

(74) Representative: **Sagittarius IP
Marlow International
Parkway
Marlow, SL7 1YL (GB)**

Remarks:
The complete document including Reference
Table(s) and the Sequence Listing(s) can be
downloaded from the EPO website

(54) **ANTI-B7-H3 ANTIBODY AND USES THEREOF**

(57) Related are a novel antibody specifically binding with B7-H3, an antigen-binding fragment of the antibody, and a composition comprising the antibody or the antigen-binding fragment thereof. Related are nucleic acids encoding the antibody or the antigen-binding fragment thereof, a host cell comprising the nucleic acids, and relevant uses. In addition, related are therapeutic and diagnostic uses of the antibody and the antigen-binding fragment thereof.

**EP 4 269 435 A1**

**Description**

**TECHNICAL FIELD**

The present invention relates to a novel antibody and an antigen-binding fragment thereof that specifically binds to B7-H3 and a composition comprising the antibody or the antigen-binding fragment thereof. In addition, the present invention relates to a nucleic acid encoding the antibody or the antigen-binding fragment thereof, a host cell comprising the nucleic acid, and related uses. In addition, the present invention relates to therapeutic and diagnostic uses of the antibody or the antigen-binding fragment thereof.

**BACKGROUND**

B7-H3 (also called CD276) is a type I transmembrane glycoprotein, which is very similar in structure to PD-L1 and belongs to the B7/CD28 superfamily. B7-H3 is widely expressed in lymphoid tissues and non-lymphoid organs at the transcription level (RNA), but the protein expression of B7-H3 is very limited, mainly expressed in activated dendritic cells, monocytes, T lymphocytes, B lymphocytes, and Nk lymphocytes, while the expression yield is very low in other normal tissues. Recently, it has been found that B7-H3 is highly expressed in a variety of solid tumors, such as lung cancer, gastric cancer, pancreatic cancer, prostate cancer, kidney cancer, ovarian cancer, endometrial cancer, colorectal cancer, liver cancer, and breast cancer, and its overexpression is closely associated with survival, prognosis, or tumor grade. In addition to being highly expressed in tumors, B7-H3 may have a function similar to PD-L1-mediated T cell inhibitory signals. It has been proposed that B7-H3 has co-stimulatory and co-inhibitory functions, depending on tumor specificity, microenvironment factors, and signal intensity. In addition to its role as an immunomodulatory agent, B7-H3 has been implicated in enhancing cancer metastasis and angiogenesis.

Since B7-H3 expression is mainly restricted to tumors, B7-H3 is a very important tumor-associated antigen that can be used as a target for potential broad-spectrum immunotherapy.

**SUMMARY**

The present invention provides an anti-B7-H3 antibody, an encoding gene thereof, and use thereof. The present invention obtains the anti-human B7-H3 antibody with high affinity and specificity of the present invention through hybridoma screening, construction of chimeric antibodies, and humanization.

In one aspect, the present invention provides a novel antibody or an antigen-binding fragment thereof that binds to B7-H3 molecules.

In some embodiments, the anti-B7-H3 antibody of the present invention has one or more of the following properties:

(i) binding to human and cynomolgus monkey B7-H3 with high affinity;
(ii) effectively binding to B7-H3 on cell surface;
(iii) effectively binding to soluble B7-H3;
(iv) effectively activating ADCC effect; and
(v) effectively inhibiting or slowing down the growth and progression of tumors *in vivo.*

In some embodiments, the anti-B7-H3 antibody or the antigen-binding fragment thereof of the present invention comprises a heavy chain variable region (VH), wherein the VH comprises

(i) three complementarity determining regions (CDRs) contained in a VH of any one of the antibodies listed in Table B; or
(ii) three heavy chain complementarity determining regions (CDRs) of any one of the antibodies listed in Table A; or
(iii) an amino acid sequence having 1 or more (preferably no more than 10, and more preferably no more than 5, 4, 3, 2, or 1) amino acid alterations (preferably amino acid replacements, and more preferably conservative amino acid replacements) compared with a VH sequence of any one of the antibodies listed in Table B, wherein the amino acid alterations do not occur in the CDRs; or
(iv) a sequence having at least 90%, 91%, 92%, 93%, 94%, 95%, 96%, 97%, 98%, 99%, or 100% identity to a VH sequence of any one of the antibodies listed in Table B, and comprising the corresponding CDR of the sequence.

In some embodiments, the anti-B7-H3 antibody or the antigen-binding fragment thereof of the present invention comprises a light chain variable region (VL), wherein the VL comprises:

(i) three complementarity determining regions (CDRs) contained in a VL of any one of the antibodies listed in Table B; or

(ii) three light chain complementarity determining regions (CDRs) of any one of the antibodies listed in Table A; or

(iii) an amino acid sequence having 1 or more (preferably no more than 10, and more preferably no more than 5, 4, 3, 2, or 1) amino acid alterations (preferably amino acid replacements, and more preferably conservative amino acid replacements) compared with a VL sequence of any one of the antibodies listed in Table B, wherein the amino acid alterations do not occur in the CDRs; or

(iv) a sequence having at least 90%, 91%, 92%, 93%, 94%, 95%, 96%, 97%, 98%, 99%, or 100% identity to a VL sequence of any one of the antibodies listed in Table B, and comprising the corresponding CDR of the sequence.

In some embodiments, the anti-B7-H3 antibody or the antigen-binding fragment thereof of the present invention comprises a heavy chain variable region VH and/or a light chain variable region VL, wherein

(a) the VH comprises:

(i) three complementarity determining regions (CDRs) contained in a VH of any one of the antibodies listed in Table B; or

(ii) three heavy chain complementarity determining regions (CDRs) of any one of the antibodies listed in Table A; or

(iii) an amino acid sequence having 1 or more (preferably no more than 10, and more preferably no more than 5, 4, 3, 2, or 1) amino acid alterations (preferably amino acid replacements, and more preferably conservative amino acid replacements) compared with a VH sequence of any one of the antibodies listed in Table B, wherein the amino acid alterations do not occur in the CDRs; or

(iv) a sequence having at least 90%, 91%, 92%, 93%, 94%, 95%, 96%, 97%, 98%, 99%, or 100% identity to a VH sequence of any one of the antibodies listed in Table B, and comprising the corresponding CDR of the sequence; and/or

(b) the VL comprises:

(i) three complementarity determining regions (CDRs) contained in a VL of any one of the antibodies listed in Table B, or

(ii) three light chain complementarity determining regions (CDRs) of any one of the antibodies listed in Table A; or

(iii) an amino acid sequence having 1 or more (preferably no more than 10, and more preferably no more than 5, 4, 3, 2, or 1) amino acid alterations (preferably amino acid replacements, and more preferably conservative amino acid replacements) compared with a VL sequence of any one of the antibodies listed in Table B, wherein the amino acid alterations do not occur in the CDRs; or

(iv) a sequence having at least 90%, 91%, 92%, 93%, 94%, 95%, 96%, 97%, 98%, 99%, or 100% identity to a VL sequence of any one of the antibodies listed in Table B, and comprising the corresponding CDR of the sequence.

In some embodiments, the present invention provides an antibody or an antigen-binding fragment thereof that binds to B7-H3, comprising: HCDR1, HCDR2 and HCDR3 sequences of a heavy chain variable region set forth in any one of SEQ ID NOs: 16, 18, 20, and 22, and/or LCDR1, LCDR2 and LCDR3 sequences of one of the light chain variable regions set forth in any one of SEQ ID NOs: 17, 19, 21, and 23, or a variant of a combination of the CDR sequences.

In other embodiments, the present invention provides an antibody or an antigen-binding fragment thereof that binds to B7-H3, which comprises 3 complementarity determining regions of a heavy chain variable region (HCDRs) and 3 complementarity determining regions of a light chain variable region (LCDRs), wherein HCDR1 comprises or consists of an amino acid sequence set forth in any one of SEQ ID NOs: 1 and 8; HCDR2 comprises or consists of an amino acid sequence set forth in any one of SEQ ID NOs: 2, 7, 9, and 14; HCDR3 comprises or consists of an amino acid sequence set forth in any one of SEQ ID NOs: 3 and 10; LCDR1 comprises or consists of an amino acid sequence set forth in any one of SEQ ID NOs: 4, 11, and 15; LCDR2 comprises or consists of an amino acid sequence set forth in any one of SEQ ID NOs: 5 and 12; and LCDR3 comprises or consists of an amino acid sequence set forth in any one of SEQ ID NOs: 6 and 13.

In some embodiments, the present invention provides an anti-B7-H3 antibody or an antigen-binding fragment thereof that binds to B7-H3 molecules, which comprises a heavy chain variable region VH and/or a light chain variable region VL, wherein

1) the VH comprises HCDR1, HCDR2 and HCDR3 contained in a VH set forth in SEQ ID NO: 16, and the VL comprises LCDR1, LCDR2 and LCDR3 contained in a VL set forth in SEQ ID NO: 17;

2) the VH comprises HCDR1, HCDR2 and HCDR3 contained in a VH set forth in SEQ ID NO: 18, and the VL comprises LCDR1, LCDR2 and LCDR3 contained in a VL set forth in SEQ ID NO: 19;

3) the VH comprises HCDR1, HCDR2 and HCDR3 contained in a VH set forth in SEQ ID NO: 20, and the VL comprises LCDR1, LCDR2 and LCDR3 contained in a VL set forth in SEQ ID NO: 21; or

4) the VH comprises HCDR1, HCDR2 and HCDR3 contained in a VH set forth in SEQ ID NO: 22, and the VL comprises LCDR1, LCDR2 and LCDR3 contained in a VL set forth in SEQ ID NO: 23.

In some embodiments, the present invention provides an anti-B7-H3 antibody or an antigen-binding fragment thereof, which comprises a heavy chain variable region VH and/or a light chain variable region VL, wherein

(i) the VH comprises HCDR1, HCDR2 and HCDR3, wherein the HCDR1 comprises or consists of an amino acid sequence set forth in any one of SEQ ID NOs: 1 and 8; the HCDR2 comprises or consists of an amino acid sequence set forth in any one of SEQ ID NOs: 2, 7, 9, and 14; the HCDR3 comprises or consists of an amino acid sequence set forth in any one of SEQ ID NOs: 3 and 10; and/or

(ii) wherein the VL comprises LCDR1, LCDR2 and LCDR3, wherein the LCDR1 comprises or consists of an amino acid sequence set forth in any one of SEQ ID NOs: 4, 11, and 15; the LCDR2 comprises or consists of an amino acid sequence set forth in any one of SEQ ID NOs: 5 and 12; the LCDR3 comprises or consists of an amino acid sequence set forth in any one of SEQ ID NOs: 6 and 13.

In some embodiments, the present invention provides an anti-B7-H3 antibody or an antigen-binding fragment thereof, which comprises:

1) HCDR1 comprising or consisting of an amino acid sequence set forth in SEQ ID NO: 1;

HCDR2 comprising or consisting of an amino acid sequence set forth in SEQ ID NO: 2;
HCDR3 comprising or consisting of an amino acid sequence set forth in SEQ ID NO: 3;
LCDR1 comprising or consisting of an amino acid sequence set forth in SEQ ID NO: 4;
LCDR2 comprising or consisting of an amino acid sequence set forth in SEQ ID NO: 5; and
LCDR3 comprising or consisting of an amino acid sequence set forth in SEQ ID NO: 6;

2) HCDR1 comprising or consisting of an amino acid sequence set forth in SEQ ID NO: 1;

HCDR2 comprising or consisting of an amino acid sequence set forth in SEQ ID NO: 7;
HCDR3 comprising or consisting of an amino acid sequence set forth in SEQ ID NO: 3;
LCDR1 comprising or consisting of an amino acid sequence set forth in SEQ ID NO: 4;
LCDR2 comprising or consisting of an amino acid sequence set forth in SEQ ID NO: 5; and
LCDR3 comprising or consisting of an amino acid sequence set forth in SEQ ID NO: 6;

3) HCDR1 comprising or consisting of an amino acid sequence set forth in SEQ ID NO: 8;

HCDR2 comprising or consisting of an amino acid sequence set forth in SEQ ID NO: 9;
HCDR3 comprising or consisting of an amino acid sequence set forth in SEQ ID NO: 10;
LCDR1 comprising or consisting of an amino acid sequence set forth in SEQ ID NO: 11;
LCDR2 comprising or consisting of an amino acid sequence set forth in SEQ ID NO: 12; and
LCDR3 comprising or consisting of an amino acid sequence set forth in SEQ ID NO: 13; or

4) HCDR1 comprising or consisting of an amino acid sequence set forth in SEQ ID NO: 8;

HCDR2 comprising or consisting of an amino acid sequence set forth in SEQ ID NO: 14;
HCDR3 comprising or consisting of an amino acid sequence set forth in SEQ ID NO: 10;
LCDR1 comprising or consisting of an amino acid sequence set forth in SEQ ID NO: 15;
LCDR2 comprising or consisting of an amino acid sequence set forth in SEQ ID NO: 12; and
LCDR3 comprising or consisting of an amino acid sequence set forth in SEQ ID NO: 13.

In some embodiments, the present invention provides an anti-B7-H3 antibody or an antigen-binding fragment thereof, which comprises a heavy chain variable region VH and/or a light chain variable region VL, wherein

(a) the heavy chain variable region VH

(i) comprises or consists of an amino acid sequence having at least 90%, 91%, 92%, 93%, 94%, 95%, 96%, 97%, 98%, or 99% identity to an amino acid sequence set forth in any one of SEQ ID NOs: 16, 18, 20, and 22, and comprises the corresponding CDR sequence of the sequence; or
(ii) comprises or consists of an amino acid sequence set forth in any one of SEQ ID NOs: 16, 18, 20, and 22; or
(iii) comprises an amino acid sequence having 1 or more (preferably no more than 10, and more preferably no more than 5, 4, 3, 2, or 1) amino acid alterations (preferably amino acid replacements, and more preferably conservative amino acid replacements) compared with an amino acid sequence set forth in any one of SEQ ID NOs: 16, 18, 20, and 22, wherein preferably, the amino acid alterations do not occur in the CDRs; and/or

(b) the light chain variable region VL

(i) comprises or consists of an amino acid sequence having at least 90%, 91%, 92%, 93%, 94%, 95%, 96%, 97%, 98%, or 99% identity to an amino acid sequence set forth in any one of SEQ ID NOs: 17, 19, 21, and 23, and comprises the corresponding CDR sequence of the sequence;
(ii) comprises or consists of an amino acid sequence set forth in any one of SEQ ID NOs: 17, 19, 21, and 23; or
(iii) comprises an amino acid sequence having 1 or more (preferably no more than 10, and more preferably no more than 5, 4, 3, 2, or 1) amino acid alterations (preferably amino acid replacements, and more preferably conservative amino acid replacements) compared with an amino acid sequence set forth in any one of SEQ ID NOs: 17, 19, 21, and 23, wherein preferably, the amino acid alterations do not occur in the CDRs.

In some embodiments, the present invention provides an anti-B7-H3 antibody or an antigen-binding fragment thereof, which comprises:

1) a heavy chain variable region VH comprising an amino acid sequence having at least 90%, 91%, 92%, 93%, 94%, 95%, 96%, 97%, 98%, or 99% identity to an amino acid sequence set forth in SEQ ID NO: 16, and a light chain variable region VL comprising an amino acid sequence having at least 90%, 91%, 92%, 93%, 94%, 95%, 96%, 97%, 98%, or 99% identity to an amino acid sequence set forth in SEQ ID NO: 17;
2) a heavy chain variable region VH comprising an amino acid sequence having at least 90%, 91%, 92%, 93%, 94%, 95%, 96%, 97%, 98%, or 99% identity to an amino acid sequence set forth in SEQ ID NO: 18, and a light chain variable region VL comprising an amino acid sequence having at least 90%, 91%, 92%, 93%, 94%, 95%, 96%, 97%, 98%, or 99% identity to an amino acid sequence set forth in SEQ ID NO: 19;
3) a heavy chain variable region VH comprising an amino acid sequence having at least 90%, 91%, 92%, 93%, 94%, 95%, 96%, 97%, 98%, or 99% identity to an amino acid sequence set forth in SEQ ID NO: 20, and a light chain variable region VL comprising an amino acid sequence having at least 90%, 91%, 92%, 93%, 94%, 95%, 96%, 97%, 98%, or 99% identity to an amino acid sequence set forth in SEQ ID NO: 21; or
4) a heavy chain variable region VH comprising an amino acid sequence having at least 90%, 91%, 92%, 93%, 94%, 95%, 96%, 97%, 98%, or 99% identity to an amino acid sequence set forth in SEQ ID NO: 22, and a light chain variable region VL comprising an amino acid sequence having at least 90%, 91%, 92%, 93%, 94%, 95%, 96%, 97%, 98%, or 99% identity to an amino acid sequence set forth in SEQ ID NO: 23.

In some embodiments, the present invention provides an antibody or an antigen-binding fragment thereof that binds to B7-H3, which comprises:

1) a heavy chain variable region VH comprising or consisting of an amino acid sequence set forth in SEQ ID NO: 16, and a light chain variable region VL comprising or consisting of an amino acid sequence set forth in SEQ ID NO: 17;
2) a heavy chain variable region VH comprising or consisting of an amino acid sequence set forth in SEQ ID NO: 18, and a light chain variable region VL comprising or consisting of an amino acid sequence set forth in SEQ ID NO: 19;
3) a heavy chain variable region VH comprising or consisting of an amino acid sequence set forth in SEQ ID NO: 20, and a light chain variable region VL comprising or consisting of an amino acid sequence set forth in SEQ ID NO: 21; or
4) a heavy chain variable region VH comprising or consisting of an amino acid sequence set forth in SEQ ID NO: 22, and a light chain variable region VL comprising or consisting of an amino acid sequence set forth in SEQ ID NO: 23.

In some embodiments, the present invention provides an anti-B7-H3 antibody or an antigen-binding fragment thereof, which comprises a heavy chain and/or a light chain, wherein

(a) the heavy chain

(i) comprises or consists of an amino acid sequence having at least 85%, 90%, 91%, 92%, 93%, 94%, 95%, 96%, 97%, 98%, or 99% identity to an amino acid sequence set forth in any one of SEQ ID NOs: 24, 26, 28, and 30, and comprising the corresponding CDR sequence of the sequence;
(ii) comprises or consists of an amino acid sequence set forth in any one of SEQ ID NOs: 24, 26, 28, and 30; or
(iii) comprises an amino acid sequence having 1 or more (preferably no more than 20 or 10, and more preferably no more than 5, 4, 3, 2, or 1) amino acid alterations (preferably amino acid replacements, and more preferably conservative amino acid replacements) compared with an amino acid sequence set forth in any one of SEQ ID NOs: 24, 26, 28, and 30, wherein preferably, the amino acid alterations do not occur in the CDRs of the heavy chain, and more preferably, the amino acid alterations do not occur in the heavy chain variable region; and/or

(b) the light chain

(i) comprises or consists of an amino acid sequence having at least 85%, 90%, 91%, 92%, 93%, 94%, 95%, 96%, 97%, 98%, or 99% identity to an amino acid sequence set forth in any one of SEQ ID NOs: 25, 27, 29, and 31, and comprising the corresponding CDR sequence of the sequence;
(ii) comprises or consists of an amino acid sequence set forth in any one of SEQ ID NOs: 25, 27, 29, and 31; or
(iii) comprises an amino acid sequence having 1 or more (preferably no more than 20 or 10, and more preferably no more than 5, 4, 3, 2, or 1) amino acid alterations (preferably amino acid replacements, and more preferably conservative amino acid replacements) compared with an amino acid sequence set forth in any one of SEQ ID NOs: 25, 27, 29, and 31, wherein preferably, the amino acid alterations do not occur in the CDRs of the light chain, and more preferably, the amino acid alterations do not occur in the light chain variable region.

In some embodiments, the present invention provides an anti-B7-H3 antibody or an antigen-binding fragment thereof, which comprises:

1) a heavy chain comprising an amino acid sequence having at least 85%, 90%, 91%, 92%, 93%, 94%, 95%, 96%, 97%, 98%, or 99% identity to an amino acid sequence set forth in SEQ ID NO: 24, and a light chain comprising an amino acid sequence having at least 85%, 90%, 91%, 92%, 93%, 94%, 95%, 96%, 97%, 98%, or 99% identity to an amino acid sequence set forth in SEQ ID NO: 25;
2) a heavy chain comprising an amino acid sequence having at least 85%, 90%, 91%, 92%, 93%, 94%, 95%, 96%, 97%, 98%, or 99% identity to an amino acid sequence set forth in SEQ ID NO: 26, and a light chain comprising an amino acid sequence having at least 85%, 90%, 91%, 92%, 93%, 94%, 95%, 96%, 97%, 98%, or 99% identity to an amino acid sequence set forth in SEQ ID NO: 27;
3) a heavy chain comprising an amino acid sequence having at least 85%, 90%, 91%, 92%, 93%, 94%, 95%, 96%, 97%, 98%, or 99% identity to an amino acid sequence set forth in SEQ ID NO: 28, and a light chain comprising an amino acid sequence having at least 85%, 90%, 91%, 92%, 93%, 94%, 95%, 96%, 97%, 98%, or 99% identity to an amino acid sequence set forth in SEQ ID NO: 29; or
4) a heavy chain comprising an amino acid sequence having at least 85%, 90%, 91%, 92%, 93%, 94%, 95%, 96%, 97%, 98%, or 99% identity to an amino acid sequence set forth in SEQ ID NO: 30, and a light chain comprising an amino acid sequence having at least 85%, 90%, 91%, 92%, 93%, 94%, 95%, 96%, 97%, 98%, or 99% identity to an amino acid sequence set forth in SEQ ID NO: 31.

In some embodiments, the present invention provides an antibody or an antigen-binding fragment thereof that binds to B7-H3, which comprises:

1) a heavy chain comprising or consisting of an amino acid sequence set forth in SEQ ID NO: 24, and a light chain comprising or consisting of an amino acid sequence set forth in SEQ ID NO: 25;
2) a heavy chain comprising or consisting of an amino acid sequence set forth in SEQ ID NO: 26, and a light chain comprising or consisting of an amino acid sequence set forth in SEQ ID NO: 27;
3) a heavy chain comprising or consisting of an amino acid sequence set forth in SEQ ID NO: 28, and a light chain comprising or consisting of an amino acid sequence set forth in SEQ ID NO: 29; or
4) a heavy chain comprising or consisting of an amino acid sequence set forth in SEQ ID NO: 30, and a light chain comprising or consisting of an amino acid sequence set forth in SEQ ID NO: 31.

In some embodiments, the anti-B7-H3 antibody of the present invention is an antibody in the form of IgG1, IgG2, IgG3, or IgG4; preferably, the anti-B7-H3 antibody is an antibody in the form of IgG1.

In some embodiments, the anti-B7-H3 antibody is a monoclonal antibody.

In some embodiments, the anti-B7-H3 antibody is a chimeric antibody. In a preferred embodiment, the anti-B7-H3 antibody is a humanized antibody. The anti-B7-H3 antibody of the present invention also encompasses an antibody fragment thereof, preferably an antibody fragment selected from: Fab, Fab', Fab'-SH, F(ab')$_2$, Fv, a single-chain antibody (e.g., scFv), a single-domain antibody, a diabody (dAb), and a linear antibody.

In some embodiments, the present invention provides an isolated nucleic acid encoding the anti-B7-H3 antibody or the antigen-binding fragment thereof, a vector comprising the nucleic acid, and a host cell comprising the nucleic acid or the vector.

In some embodiments, the present invention provides a method for preparing the anti-B7-H3 antibody or the antigen-binding fragment thereof, wherein the method comprises culturing the host cell described herein under conditions suitable for expressing a nucleic acid encoding the antibody or the antigen-binding fragment thereof described herein. In another embodiment, the present invention provides an anti-B7-H3 antibody and an antigen-binding fragment thereof prepared by the method described above.

In some embodiments, the present invention provides an immunoconjugate and a pharmaceutical composition comprising the anti-B7-H3 antibody or the antigen-binding fragment thereof.

In some embodiments, the present invention also provides use of the anti-B7-H3 antibody or the antigen-binding fragment thereof, the immunoconjugate, or the pharmaceutical composition in the preparation of a medicament for the prevention and/or treatment of a B7-H3-related disease or disorder (e.g., a tumor).

In some embodiments, the present invention also provides a method for preventing and/or treating a B7-H3-related disease or disorder (e.g., a tumor), which comprises administering to a subject an effective amount of the antibody or the antigen-binding fragment thereof that binds to B7-H3, the immunoconjugate, or the pharmaceutical composition of the present invention.

The present invention also relates to a method for detecting a B7-H3 molecule in a sample, which comprises (a) contacting the antibody or the antigen-binding fragment thereof described herein with the sample; and (b) detecting whether a complex is formed by the antibody or the antigen-binding fragment thereof and the B7-H3 molecule in the sample.

In another aspect, the present invention also relates to a method for diagnosing a tumor expressing a B7-H3 molecule in a subject, which comprises (a) obtaining a sample from the subject; (b) contacting the antibody or the antigen-binding fragment thereof described herein with the sample; and (c) detecting whether a complex is formed by the antibody or the antigen-binding fragment thereof and the B7-H3 molecule in the sample.

## BRIEF DESCRIPTION OF THE DRAWINGS

The preferred embodiments of the present invention described in detail below will be better understood when read in conjunction with the following drawings. For the purpose of illustrating the present invention, currently preferred embodiments are shown in the drawings. However, it should be understood that the present invention is not limited to an accurate arrangement and means of the embodiments shown in the drawings.

FIG. 1 shows assay results of the antibodies binding to CHOS cells overexpressing human B7H3 by FACS.

FIG. 2 shows assay results of ADCC activity of the antibodies.

FIG. 3 shows *in vivo* anti-tumor results of the antibodies, wherein FIG. 3a shows tumor volume changes in tumor-bearing mice, and FIGs. 3b-3c show body weight changes in tumor-bearing mice.

## DETAILED DESCRIPTION

### I. Definitions

Before the present invention is described in detail below, it should be understood that the present invention is not limited to the particular methodology, protocols, and reagents described herein, as these may vary. It should also be understood that the terminology used herein is only intended to describe specific embodiments rather than limit the scope of the present invention, which will be limited only by the appended claims. Unless otherwise defined, any technical and scientific term used herein has the same meaning as commonly understood by those of ordinary skill in the art to which the present invention belongs.

For the purpose of explaining this specification, the following definitions will be used, and wherever appropriate, terms used in the singular form may also include the plural form, and vice versa. It should be understood that the terms used herein are for the purpose of describing specific embodiments only, and are not intended to be limiting. The term "about" used in combination with a numerical value is intended to encompass the numerical values in a range from a lower limit 5% lower than the specified numerical value to an upper limit 5% higher than the specified numerical value.

The term "and/or" means that when used to connect two or more options, it should be understood to refer to any one of the options or any two or more of the options.

The term "comprise" or "include" means that the described elements, integers or steps are included, but not to the exclusion of any other elements, integers or steps. The term "comprise" or "include" used herein, unless otherwise specified, also encompasses the situation where the entirety consists of the described elements, integers or steps. For example, when referring to an antibody variable region "comprising" a particular sequence, it is also intended to encompass an antibody variable region consisting of the particular sequence.

The term "antibody" is used herein in the broadest sense and encompasses a variety of antibody structures, including but not limited to, a monoclonal antibody, a polyclonal antibody, a recombinant antibody, a humanized antibody, a chimeric antibody, a multispecific antibody (e.g., a bispecific antibody), a single-chain antibody, an intact antibody, or an antibody fragment thereof that exhibits the desired antigen-binding activity. An intact antibody will generally comprise at least two full-length heavy chains and two full-length light chains, but may comprise fewer chains in some cases, for example, natural antibodies in a camel may only comprise heavy chains. The term "antigen-binding fragment" (used interchangeably herein with "antibody fragment" and "antigen-binding portion") refers to a molecule different from an intact antibody, which comprises a portion of the intact antibody and binds to an antigen to which the intact antibody binds. Examples of antigen-binding fragments include, but are not limited to, Fv, Fab, Fab', Fab'-SH, F(ab')$_2$; diabodies (dAbs); linear antibodies; single-chain antibodies (e.g., scFvs); single-domain antibodies; antigen-binding fragments of bivalent or bispecific antibodies; camelid antibodies; and other fragments that exhibit the desired ability to bind to an antigen (e.g., B7-H3).

"Affinity" or "binding affinity" refers to inherent binding affinity that reflects the interaction between members of a binding pair. The affinity of molecule X for its partner Y can be generally represented by the equilibrium dissociation constant (KD), which is a ratio of the dissociation rate constant to the association rate constant (kdis and kon, respectively). The affinity can be measured by common methods known in the art. One particular method for measuring the affinity is the ForteBio kinetic binding assay herein.

The term "Fc region" is used herein to define a C-terminus region of an immunoglobulin heavy chain, which comprises at least one portion of a constant region. The "Fc region" includes Fc regions of native sequences and variant Fc regions. In certain embodiments, a human IgG heavy chain Fc region generally extends from Cys226 or Pro230 to a carbonyl terminus of a heavy chain. However, the C-terminal lysine (Lys447) of the Fc region may or may not be present. Unless otherwise stated, amino acid residues in the Fc region or constant region are numbered according to the EU numbering system, also called the EU index, as described in Kabat et al., Sequences of Proteins of Immunological Interest, 5th Ed. Public Health Service, National Institutes of Health, Bethesda, MD, 1991.

The term "variable region" or "variable domain" refers to a domain of a heavy chain or light chain of an antibody involved in the binding of the antibody to an antigen. Variable domains of heavy and light chains of natural antibodies typically have similar structures, wherein each domain comprises four conserved framework regions (FRs) and three complementarity determining regions (see, e.g., Kindt et al., Kuby Immunology, 6th ed., W. H. Freeman and Co., page 91 (2007)). A single VH or VL domain may be sufficient to provide antigen-binding specificity. In addition, libraries of complementary VL or VH domains can be screened using a VH or VL domain from an antibody that binds to a particular antigen to isolate antibodies that bind to the antigen (see, e.g., Portolano et al., J. Immunol., 150: 880-887 (1993); Clarkson et al., Nature, 352: 624-628 (1991)).

"Complementarity determining region" or "CDR region" or "CDR" or "highly variable region" (used interchangeably herein with hypervariable region "HVR") is a region in an antibody variable domain that is highly variable in sequence and forms a structurally defined loop ("hypervariable loop") and/or comprises antigen-contacting residues ("antigen contact site"). CDRs are primarily responsible for binding to antigen epitopes. The CDRs of heavy and light chains are numbered sequentially from the N-terminus and are generally referred to as CDR1, CDR2, and CDR3. The CDRs located in a heavy chain variable domain of an antibody are also referred to as HCDR1, HCDR2, and HCDR3, whereas the CDRs located in a light chain variable domain of an antibody are referred to as LCDR1, LCDR2, and LCDR3. In a given amino acid sequence of a light chain variable region or a heavy chain variable region, the CDR sequences can be determined using a variety of schemes well known in the art, for example, Kabat complementarity determining regions (CDRs) are determined based on sequence variability and are most commonly used (Kabat et al., Sequences of Proteins of Immunological Interest, 5th Ed., Public Health Service, National Institutes of Health, Bethesda, Md. (1991)). Chothia scheme is based on the positions of structural loops (Chothia and Lesk, J. mol. biol. 196:901-917 (1987)). AbM CDRs are a compromise between Kabat CDRs and Chothia structural loops and are used by Oxford Molecular's AbM antibody modeling software. The "contact" CDRs are based on the analysis of available complex crystal structures. According to different CDR determination schemes, the residue of each HVR/CDR among these CDRs is described as follows.

| CDR | Kabat scheme | AbM scheme | Chothia scheme | Contact scheme |
|---|---|---|---|---|
| LCDR1 | L24-L34 | L24-L34 | L26-L32 | L30-L36 |
| LCDR2 | L50-L56 | L50-L56 | L50-L52 | L46-L55 |
| LCDR3 | L89-L97 | L89-L97 | L91-L96 | L89-L96 |
| HCDR1 | H31-H35B | H26-H35B | H26-H32 | H30-H35B |
| (Kabat numbering system) | | | | |
| HCDR1 | H31-H35 | H26-H35 | H26-H32 | H30-H35 |
| (Chothia numbering system) | | | | |
| HCDR2 | H50-H65 | H50-H58 | H53-H55 | H47-H58 |
| HCDR3 | H95-H102 | H95-H102 | H96-H101 | H93-H101 |
| (Kabat numbering system) | | | | |

CDRs can also be CDR sequences located at the following Kabat residue positions according to the Kabat numbering system:

positions 24-36 or 24-34 (LCDR1), positions 46-56 or 50-56 (LCDR2), and positions 89-97 or 89-96 (LCDR3) in VL; and positions 26-35 or 27-35B (HCDR1), positions 50-65 or 49-65 (HCDR2), and positions 93-102, 94-102, or 95-102 (HCDR3) in VH.

In one embodiment, the boundary of the HCDR1 of the antibody of the present invention is determined by AbM scheme, and the boundaries of the HCDR2, HCDR3 and LCDRs are determined by Kabat scheme, e.g., as shown in Table A below.

CDRs can also be determined based on having the same Kabat numbering positions as a reference CDR sequence (e.g., any of the exemplary CDRs of the present invention).

Unless otherwise stated, residue positions of an antibody variable region (including heavy chain variable region residues and light chain variable region residues) are numbered according to the Kabat numbering system (Kabat et al., Sequences of Proteins of Immunological Interest, 5th Ed., Public Health Service, National Institutes of Health, Bethesda, Md. (1991)) herein.

Unless otherwise stated, the term "CDR" or "CDR sequence" used herein encompasses CDR sequences determined by any one of the schemes above.

However, it should be noted that boundaries of the CDRs of variable regions of the same antibody based on different assignment systems may differ. That is, the CDR sequences of variable regions of the same antibody defined by different assignment systems differ. Accordingly, when it comes to defining an antibody with specific CDR sequences defined in the present invention, the scope of the antibody also encompasses such antibodies whose variable region sequences comprise the specific CDR sequences, but have claimed CDR boundaries different from the specific CDR boundaries defined by the present invention due to a different protocol (e.g., different assignment system rules or their combinations) applied.

Antibodies with different specificities (i.e., different binding sites for different antigens) have different CDRs. However, although CDRs differ from antibody to antibody, only a limited number of amino acid positions within the CDRs are directly involved in antigen binding. The smallest overlapping region can be determined using at least two of the Kabat, Chothia, AbM, Contact, and North schemes, thereby providing a "minimal binding unit" for antigen binding. The minimal binding unit may be a sub-portion of the CDR. As will be clear to those skilled in the art, residues in remaining portions of the CDR sequences can be determined by the structure and protein folding of the antibody. Accordingly, variants of any CDR presented herein are also contemplated by the present invention. For example, in a variant of one CDR, the amino acid residue of the minimal binding unit may remain unchanged, while the remaining CDR residues defined according to Kabat or Chothia may be substituted by conservative amino acid residues.

The term "antibody-dependent cell-mediated cytotoxicity" or "ADCC" refers to a form of cytotoxicity in which secreted immunoglobulins binding to Fc receptors (FcRs) present on certain cytotoxic cells (e.g., NK cells, neutrophils, and macrophages) enable these cytotoxic effector cells to specifically bind to an antigen-bearing target cell and subsequently kill the target cell with cytotoxins. The primary cells mediating ADCC, NK cells, express FcγRIII only, whereas monocytes express FcγRI, FcγRII, and FcγRIII. An *in vitro* ADCC assay can be performed to assess ADCC activity of a molecule of interest, or the ADCC activity of the molecule of interest can be assessed *in vivo*, e.g., in an animal model. An exemplary assay for assessing the ADCC activity is provided in the examples herein.

The term "functional Fc region" refers to an Fc region that possesses the "effector functions" of Fc regions of native sequences. Exemplary "effector functions" include C1q binding, CDC, Fc receptor binding, ADCC, phagocytosis, cell surface receptor (e.g., B cell receptor, or BCR) down-regulation, and the like. Such effector functions generally require that the Fc region is associated with a binding domain (e.g., an antibody variable domain) and can be assessed using a variety of assays, such as those disclosed herein.

The term "therapeutic agent" described herein encompasses any substance effective in preventing or treating tumors (e.g., cancer), including chemotherapeutic agents, cytotoxic agents, vaccines, other antibodies, anti-infective active agents, small molecule drugs, or immunomodulatory agents.

The term "immunomodulatory agent" used herein refers to a natural or synthetic active agent or drug that suppresses or modulates an immune response. The immune response may be a humoral response or a cellular response.

The term "effective amount" refers to an amount or dosage of the antibody, fragment thereof, conjugate or composition of the present invention which generates expected effects in a patient in need of treatment or prevention after administration to the patient in a single or multiple doses. For therapeutic or prophylactic purposes, the "effective amount" can be divided into a "therapeutically effective amount" and a "prophylactically effective amount". The effective amount can be easily determined by an attending physician as a person skilled in the art by considering a variety of factors as follows: species such as mammals, size, age, general health condition, the specific disease involved, the extent or severity of the disease, response in an individual patient, specific antibody administered, mode of administration, bioavailability characteristics of the administered formulation, selected administration regimen, and use of any concomitant therapy.

In one embodiment, an effective amount of the B7-H3 antibody of the present invention preferably inhibits a measurable parameter (e.g., tumor growth rate, tumor volume, etc.) by at least about 20%, and more preferably at least about 40%, relative to a control.

The terms "host cell", "host cell line" and "host cell culture" are used interchangeably and refer to cells into which exogenous nucleic acids are introduced, including progenies of such cells. Host cells include "transformants" and "transformed cells", which include original primary transformed cells and progenies derived therefrom, regardless of the number of passages. Progeny may not be exactly the same as parent cells in terms of nucleic acid content, and may contain mutations. Mutant progeny having the same function or biological activities that are screened or selected from the initially transformed cells are included herein.

The term "chimeric antibody" refers to an antibody in which the variable region sequences are derived from one species and the constant region sequences are derived from another species, e.g., an antibody in which the variable region sequences are derived from a mouse antibody and the constant region sequences are derived from a human antibody.

The term "humanized antibody" refers to an antibody in which antigen-binding sites derived from another mammalian species, such as mice, are linked to human immunoglobulin sequences. A humanized antibody is a chimeric molecule, typically prepared using recombinant techniques, in which additional framework region modifications can be introduced within the human framework sequences. The antigen-binding site may comprise an entire variable domain fused to a constant region, or only comprise complementarity determining regions grafted to an appropriate framework sequence in the variable domain. In some embodiments, a humanized antibody will comprise at least one, or generally two of substantially all variable domains in which all or substantially all CDRs (e.g., 6 CDRs) correspond to those of a non-human antibody, and all or substantially all FRs correspond to those of a human antibody. A humanized antibody may optionally comprise at least a portion of an antibody constant region derived from a human antibody. The "humanized form" of an antibody (such as a non-human antibody) refers to an antibody that has been humanized.

The term "immunoconjugate" is an antibody conjugated to one or more other substances, including but not limited to cytotoxic agents or labels.

The term "label" used herein refers to a compound or composition which is directly or indirectly conjugated or fused to an agent, such as a polynucleotide probe or an antibody, and facilitates the detection of the agent to which it is conjugated or fused. The label itself can be detectable (e.g., a radioisotope label or a fluorescent label) or can catalyze a chemical change to a detectable substrate compound or composition in the case of enzymatic labeling. The term is intended to encompass direct labeling of a probe or an antibody by coupling (i.e., physical linking) a detectable substance to the probe or an antibody and indirect labeling of a probe or antibody by reacting with another reagent which is directly labeled. Examples of indirect labeling include detection of a primary antibody using a fluorescently labeled secondary antibody, and end labeling of a biotinylated DNA probe, such that it can be detected with a fluorescently labeled streptavidin.

The term "individual" or "subject" includes mammals. The mammals include, but are not limited to, domestic animals (e.g., cattle, goats, cats, dogs, and horses), primates (e.g., human and non-human primates such as monkeys), rabbits, and rodents (e.g., mice and rats). In some embodiments, the individual or subject is a human. The term "isolated" antibody is an antibody which has been separated from components of its natural environment. In some embodiments, the antibody is purified to a purity greater than 95% or 99% as determined by, e.g., electrophoresis (e.g., SDS-PAGE, isoelectric focusing (IEF) and capillary electrophoresis) or chromatography (e.g., ion exchange or reverse-phase HPLC).

"An isolated nucleic acid encoding an anti-B7-H3 antibody or an antigen-binding fragment thereof" refers to one or more nucleic acid molecules encoding a heavy chain or a light chain of the antibody (or the antigen-binding fragment thereof), including such nucleic acid molecules in a single vector or separated vectors, and such nucleic acid molecules present at one or more positions in a host cell.

The calculation of sequence identity between sequences is performed as follows.

To determine the percent identity of two amino acid sequences or two nucleic acid sequences, the sequences are aligned for optimal comparison purposes (e.g., for optimal alignment, gaps can be introduced in one or both of the first and second amino acid sequences or nucleic acid sequences, or non-homologous sequences can be discarded for comparison). In one preferred embodiment, for comparison purposes, the length of the aligned reference sequence is at least 30%, preferably at least 40%, more preferably at least 50% or 60%, and even more preferably at least 70%, 80%, 90%, or 100% of the length of the reference sequence. Amino acid residues or nucleotides at corresponding amino acid positions or nucleotide positions are then compared. When a position in the first sequence is occupied by the same amino acid residue or nucleotide at the corresponding position in the second sequence, the molecules are identical at this position.

A mathematical algorithm can be used to compare two sequences and calculate the percent identity between the sequences. In one preferred embodiment, the percent identity between two amino acid sequences is determined with the Needlema and Wunsch algorithm ((1970) J. Mol. Biol., 48:444-453; available at http://www.gcg.com) which has been integrated into the GAP program of the GCG software package, using the Blossom 62 matrix or PAM250 matrix and gap weight of 16, 14, 12, 10, 8, 6, or 4 and length weight of 1, 2, 3, 4, 5, or 6. In another preferred embodiment, the percent identity between two nucleotide acid sequences is determined with the GAP program of the GCG software package (available at http://www.gcg.com), using the NWSgapdna.CMP matrix and gap weight of 40, 50, 60, 70, or 80 and length weight of 1, 2, 3, 4, 5, or 6. A particularly preferred parameter set (and one that should be used unless otherwise stated) is a Blossom 62 scoring matrix with a gap penalty of 12, a gap extension penalty of 4, and a frameshift gap penalty of 5.

The percent identity between two amino acid sequences or nucleotide sequences can also be determined with PAM120 weighted remainder table, a gap length penalty of 12 and a gap penalty of 4, using the E. Meyers and W Miller algorithm ((1989) CABIOS, 4:11-17) which has been incorporated into the ALIGN program (version 2.0).

Additionally or alternatively, the nucleic acid sequences and protein sequences described herein can be further used as "query sequences" to perform searches against public databases to, e.g., identify other family member sequences or related sequences.

The term "pharmaceutical supplementary material" refers to diluents, adjuvants (e.g., Freund's adjuvants (complete and incomplete)), excipients, carriers, stabilizers, or the like, which are administered with the active substance. The term "pharmaceutical composition" refers to such a composition that exists in a form allowing effective biological activity of the active ingredient contained therein, and does not contain additional ingredients having unacceptable toxicity to a subject to which the composition is administered.

As used herein, "treatment" (or "treat" or "treating") refers to slowing, interrupting, arresting, alleviating, stopping, lowering, or reversing the progression or severity of an existing symptom, disorder, condition, or disease.

As used herein, "prevention" (or "prevent" or "preventing") includes the inhibition of the onset or progression of symptoms of a disease or disorder, or a specific disease or disorder. In some embodiments, subjects with family history of cancer are candidates for preventive regimens. Generally, in the context of cancer, the term "prevention" refers to the administration of a drug prior to the onset of signs or symptoms of cancer, particularly in subjects at risk of cancer.

The term "vector" used herein refers to a nucleic acid molecule capable of proliferating another nucleic acid to which it is linked. The term includes vectors that serve as self-replicating nucleic acid structures as well as vectors binding to the genome of a host cell into which they have been introduced. Some vectors are capable of directing the expression of a nucleic acid to which they are operably linked. Such vectors are called "expression vectors" herein.

The term "subject/patient sample" refers to a collection of tissue or cell samples obtained from a patient or a subject. The source of tissue or cell samples can be solid tissues, e.g., from fresh, frozen and/or preserved organ or tissue samples or biopsy samples or puncture samples; blood or any blood component; body fluids such as cerebrospinal fluids, amniotic fluids, peritoneal fluids, or interstitial fluids; and cells from a subject at any time during pregnancy or development.

## II. Antibody

Unless otherwise stated, the terms "B7-H3", "B7H3", and "CD276" are used interchangeably herein. B7-H3 is a type I transmembrane glycoprotein, which is a member of the B7/CD28 superfamily, and is similar in sequence to the extracellular domain of PD-L1. B7-H3 has 316 amino acids and comprises a putative signal peptide consisting of 28 amino acids, an extracellular region consisting of 217 amino acids, a transmembrane region, and a cytoplasmic domain consisting of 45 amino acids, with a molecular weight of about 45-66 kDa. In humans, the extracellular structure of B7-H3 may be an IgV-IgC-like domain (2Ig-B7-H3) or an IgV-IgC-IgV-IgC-like domain (4Ig-B7-H3) due to exon duplication. The sequence of cynomolgus monkey B7-H3 has about 90% homology to its human counterpart.

The term "anti-B7-H3 antibody", "anti-B7-H3", "B7-H3 antibody", or "antibody against B7-H3" as used herein refers to an antibody or an antigen-binding fragment thereof capable of binding to B7-H3 protein with sufficient affinity. The antibody may be used as a diagnostic agent and/or a therapeutic agent in targeting B7-H3.

In some embodiments, the anti-B7-H3 antibody or the antigen-binding fragment thereof of the present invention binds to B7-H3 (e.g., human or cynomolgus monkey B7-H3) with sufficient affinity, for example, binds to B7-H3 with an equilibrium dissociation constant ($K_D$) of $\leq 1\ \mu M$, $\leq 100\ nM$, $\leq 10\ nM$, $\leq 1\ nM$, $\leq 0.1\ nM$, $\leq 0.01\ nM$, or $\leq 0.001\ nM$ (e.g., $10^{-7}$ M or less, such as $10^{-7}$ M to $10^{-10}$ M). In some embodiments, the B7-H3 is a human or cynomolgus monkey B7-H3. In some embodiments, the antibody binding affinity is determined using biological optical interferometry, for example, in biological optical interferometry, an antibody binds to human B7-H3 with a $K_D$ of about $1 \times 10^{-7}$ M or less, about $5 \times 10^{-8}$ M or less, about $1 \times 10^{-8}$ M or less, or about $5 \times 10^{-9}$ M or less, a $K_D$ of about $1 \times 10^{-9}$ M or less, or a $K_D$ of about $1 \times 10^{-10}$ M or less.

In some embodiments, the antibody or the antigen-binding fragment thereof of the present invention binds to B7-H3 expressed on cell surface.

In some embodiments, the antibody or the antigen-binding fragment thereof of the present invention can induce an ADCC effect. In some embodiments, the antibody or the antigen-binding fragment thereof of the present invention can inhibit and/or reduce the growth and/or volume of a tumor *in vivo.*

In some embodiments, the antibody or the antigen-binding fragment thereof that binds to B7-H3 of the present invention comprises a heavy chain variable region (VH) and/or a light chain variable region (VL), wherein the VH and VL comprise combinations of 6 CDRs selected from Table A.

In one embodiment of the present invention, the amino acid alteration described herein includes amino acid replacement, insertion or deletion. Preferably, the amino acid alteration described herein is an amino acid replacement, preferably a conservative replacement.

In a preferred embodiment, the amino acid alteration described herein occurs in a region outside the CDR (e.g., in FR). More preferably, the amino acid alteration described herein occurs in a region outside the heavy chain variable region and/or outside the light chain variable region.

In some embodiments, the replacement is a conservative replacement. A conservative replacement refers to the replacement of an amino acid by another amino acid of the same class, e.g., the replacement of an acidic amino acid by another acidic amino acid, the replacement of a basic amino acid by another basic amino acid, or the replacement of a neutral amino acid by another neutral amino acid. Exemplary replacements are shown in the table below:

| Original residue | Exemplary replacement | Preferred conservative amino acid replacement |
| --- | --- | --- |
| Ala (A) | Val, Leu, Ile | Val |
| Arg (R) | Lys, Gln, Asn | Lys |
| Asn (N) | Gln, His, Asp, Lys, Arg | Gin |
| Asp (D) | Glu, Asn | Glu |
| Cys (C) | Ser, Ala | Ser |
| Gln (Q) | Asn, Glu | Asn |
| Glu (E) | Asp, Gln | Asp |
| Gly (G) | Ala | Ala |
| His (H) | Asn, Gln, Lys, Arg | Arg |
| Ile (I) | Leu, Val, Met, Ala, Phe, Nle | Leu |
| Leu (L) | Nle, Ile, Val, Met, Ala, Phe | Ile |
| Lys (K) | Arg, Gln, Asn | Arg |
| Met (M) | Leu, Phe, Ile | Leu |
| Phe (F) | Trp, Leu, Ual, Ile, Ala, Tyr | Tyr |
| Pro (P) | Ala | Ala |
| Ser (S) | Thr | Thr |
| Thr (T) | Val, Ser | Ser |

(continued)

| Original residue | Exemplary replacement | Preferred conservative amino acid replacement |
|---|---|---|
| Tip (W) | Tyr, Phe | Tyr |
| Tyr (Y) | Trp, Phe, Thr, Ser | Phe |
| Val (V) | Ile, Leu, Met, Phe, Ala, Nle | Leu |

In certain embodiments, the replacement occurs in the CDRs of the antibody. Generally, the obtained variant has modifications (e.g., improvements) in certain biological properties (e.g., increased affinity) relative to the parent antibody and/or will substantially retain certain biological properties of the parent antibody. Exemplary replacement variants are affinity-matured antibodies.

In certain embodiments, the antibody provided herein is altered to increase or decrease the extent to which the antibody is glycosylated. Addition or deletion of glycosylation sites of an antibody can be conveniently achieved by altering the amino acid sequence to create or remove one or more glycosylation sites. When the antibody comprises an Fc region, carbohydrate attached thereto can be altered. In some applications, removing undesired modifications to glycosylation sites is useful, for example, removing fucose modules to enhance antibody-dependent cell-mediated cytotoxicity (ADCC) (see Shield et al., (2002) *JBC,* 277:26733). In other applications, galactosidylation modification can be carried out to modify complement-dependent cytotoxicity (CDC).

In certain embodiments, one or more amino acid modifications may be introduced into an Fc region of an antibody provided herein, thus producing an Fc region variant. The Fc region variant may comprise a human Fc region sequence (such as human IgG1, IgG2, IgG3, or IgG4 Fc region) comprising an amino acid modification (such as replacement) at one or more amino acid positions. For examples of the Fc variant, see U.S. Patent No. 7,332,581, U.S. Patent No. 6,737,056, U.S. Patent No. 6,737,056; WO 2004/056312 and Shields et al., J. Biol. Chem., 9(2):6591-6604(2001), U.S. Patent No. 6,194,551, WO 99/51642 and Idusogie et al., J. Immunol., 164:4178-4184 (2000), U.S. Patent No. 7,371,826, Duncan & Winter, Nature 322:738-40 (1988); U.S. Patent No. 5,648,260; U.S. Patent No. 5,624,821; and WO 94/29351.

In certain embodiments, antibodies modified by cysteine engineering may need to be produced, such as "sulfo-MAb", wherein one or more residues of the antibodies are replaced by cysteine residues. A cysteine-modified antibody can be produced as described, for example, in U.S. Patent No. 7,521,541.

In certain embodiments, the antibody provided herein can be further modified to comprise other non-protein portions known in the art and readily available. Suitable portions for antibody derivatization include, but are not limited to, water-soluble polymers. Non-limiting examples of water-soluble polymers include, but are not limited to, polyethylene glycol (PEG), ethylene glycol/propylene glycol copolymer, carboxymethyl cellulose, glucan, polyvinyl alcohol, polyvinylpyrrolidone, poly-1,3-dioxane, poly-1,3,6-trioxane, ethylene/maleic anhydride copolymer, polyamino acid (homopolymer or random copolymer), and glucan or poly(n-vinylpyrrolidone)polyethylene glycol, propylene glycol homopolymer, polypropylene oxide/ethylene oxide copolymer, polyoxyethylated polyol (e.g., glycerol), polyvinyl alcohol and mixtures thereof.

### III. Nucleic acid of the present invention, and vector and host cell comprising same

The present invention provides a nucleic acid encoding any of the above anti-B7-H3 antibodies or antigen-binding fragments thereof, and also provides a vector comprising the nucleic acid. In one embodiment, the vector is an expression vector.

The present invention also provides a host cell comprising the nucleic acid or the vector. In one embodiment, the host cell is eukaryotic. In another embodiment, the host cell is selected from an *E. coli* cell, a mammal cell (e.g., CHO cell or 293 cell), and other cells suitable for preparing an antibody or an antigen-binding fragment thereof. In another embodiment, the host cell is prokaryotic. In one embodiment, the host cell is selected from an *E. coli* cell.

As will be appreciated by those skilled in the art, each antibody or polypeptide amino acid sequence may be encoded by a variety of nucleic acid sequences because of codon degeneracy.

These polynucleotide sequences can be generated by *de novo* solid phase DNA synthesis or by PCR mutagenesis of sequences encoding the antibody or the antigen-binding fragment thereof that binds to B7-H3 using methods well known in the art.

In one embodiment, one or more vectors comprising the nucleic acid of the present invention are provided. In one embodiment, the vector is an expression vector, such as a eukaryotic expression vector. The vector includes, but is not limited to, a virus, a plasmid, a cosmid, a λ phage, or a yeast artificial chromosome (YAC).

In one embodiment, provided is a host cell comprising the vector. The suitable host cell for cloning or expressing the vector encoding the antibody includes prokaryotic cells or eukaryotic cells described herein. For example, the antibody may be produced in bacteria, particularly when glycosylation and Fc effector functions are not required. Expression of antibody fragments and polypeptides in bacteria is described in, for example, U.S. Pat. Nos. 5,648,237, 5,789,199 and 5,840,523, and also described in Charlton, Methods in Molecular Biology, Vol. 248 (B. K. C. Lo, Ed., Humana Press, Totowa, NJ, 2003), pg. 245-254, which describes expression of antibody fragments in *E. coli*. After expression, the antibody can be isolated from bacterial cell paste in soluble fraction and can be further purified.

In one embodiment, the host cell is eukaryotic. In another embodiment, the host cell is selected from a yeast cell, a mammalian cell, and other cells suitable for preparing an antibody or an antigen-binding fragment thereof. For example, eukaryotic microorganisms, such as filamentous fungi or yeast, are suitable cloning or expressing hosts for the vector encoding the antibody. For example, fungus and yeast strains in which the glycosylation pathway has been "humanized" may produce antibodies having a partial or full human glycosylation pattern. See Gerngross, Nat. Biotech., 22:1409-1414 (2004), and Li et al., Nat. Biotech., 24:210-215 (2006). Host cells suitable for expressing glycosylated antibodies are also derived from multicellular organisms (invertebrates and vertebrates). Vertebrate cells may also be used as hosts. For example, a mammalian cell line engineered to be suitable for suspension growth may be used. Other examples of useful mammalian host cell lines are monkey kidney CV1 lines (COS-7) transformed with SV40, human embryonic kidney lines (293HEK or 293 cells, as described in, e.g., Graham et al., J. Gen Virol. 36: 59 (1977)) and the like. Other useful mammalian host cell lines include Chinese hamster ovary (CHO) cells, including DHFR-CHO cells (Urlaub et al., Proc. Natl. Acad. Sci. USA 77: 216 (1980)), and myeloma cell lines such as Y0, NS0, and Sp2/0. For reviews of certain mammalian host cell lines suitable for antibody production, see, e.g., Yazaki and Wu, Methods in Molecular Biology, Vol. 248 (B. K. C. Lo, ed., Humana Press, Totowa, NJ), pg. 255-268 (2003).

### IV. Production and purification of the antibody molecule of the present invention

In one embodiment, the present invention provides a method for preparing an anti-B7-H3 antibody or a fragment thereof (preferably an antigen-binding fragment), wherein the method comprises culturing the host cell under conditions suitable for expressing a nucleic acid encoding the antibody or the fragment thereof (preferably the antigen-binding fragment), and optionally isolating the antibody or the fragment thereof. In a certain embodiment, the method further comprises recovering the anti-B7-H3 antibody or the fragment thereof from the host cell.

### V. Multispecific antibody

In a further aspect, the present invention provides a multispecific (including bispecific) antibody molecule that specifically binds to B7-H3. In one embodiment, in the multispecific antibody, the antibody (or the antigen-binding fragment thereof) of the present invention has a first binding specificity for B7-H3.

In one embodiment, the binding specificity is dependent on a "binding site" or an "antigen-binding site" (a region of an antibody molecule that actually binds to an antigen) of the antibody. In a preferred embodiment, the antigen-binding site is formed by a VH/VL pair consisting of a light chain variable domain (VL) and a heavy chain variable domain (VH) of the antibody. Accordingly, in one embodiment, the "multispecific" antibody is an antibody having at least two antigen-binding sites, each of which can bind to a different epitope of the same antigen or a different epitope of a different antigen.

For multispecific antibodies and preparation thereof, see, for example, the descriptions in WO 2009/080251, WO 2009/080252, WO 2009/080253, WO 2009/080254, WO 2010/112193, WO 2010/115589, WO 2010/136172, WO 2010/145792, and WO 2010/145793.

### VI. Immunoconjugate

In some embodiments, the present invention provides an immunoconjugate produced by conjugating the antibody of the present invention to a heterologous molecule. In some embodiments, the heterologous molecule is, for example, a therapeutic agent or a diagnostic agent, such as a cytotoxic agent or a chemotherapeutic agent. The cytotoxic agent includes any agent that is harmful to cells. Examples of the cytotoxic agent suitable for forming the immunoconjugate are known in the art.

Linkers can be used to covalently link different entities of the conjugates. Suitable linkers include chemical linkers or peptide linkers. Advantageously, the linkers are "cleavable linkers" that facilitate the release of the polypeptides following delivery to a target site. For example, acid-labile linkers, peptidase-sensitive linkers, photolabile linkers, dimethyl linkers or disulfide-containing linkers can be used (Chari et al., Cancer Research, 52 (1992) 127-131; US 5,208,020).

In some embodiments, the antibody of the present invention can be conjugated to a diagnostic agent or a detectable agent. Such conjugates can be used as part of a clinical testing method (e.g., to determine the efficacy of a particular therapy) to monitor or predict the onset, formation, progression, and/or severity of a disease or disorder. Such diagnosis and detection can be achieved by coupling the antibody to the detectable agent, which includes, but is not limited to, a variety of enzymes, such as but not limited to, horseradish peroxidase; prosthetic groups, such as but not limited to, streptavidin/biotin and avidin/biotin; fluorescent substances; luminescent substances; radioactive substances; and positron-emitting metals and non-radioactive paramagnetic metal ions used in various positron emission tomography techniques.

In some embodiments, the immunoconjugate is used to prevent or treat a tumor. In some embodiments, the tumor is cancer.

## VII. Pharmaceutical composition, pharmaceutical formulation, and combination product

The present invention further comprises a composition (including a pharmaceutical composition or a pharmaceutical formulation) comprising the anti-B7-H3 antibody or the immunoconjugate thereof or the multispecific antibody, and a composition comprising a polynucleotide encoding the anti-B7-H3 antibody or the immunoconjugate thereof or the multispecific antibody. Such compositions can further optionally comprise a suitable pharmaceutical supplementary material, such as a pharmaceutical carrier and a pharmaceutical excipient known in the art, including buffers.

The pharmaceutical composition or formulation of the present invention can also be combined with one or more other active ingredients which are required for a specific indication being treated, preferably active ingredients having complementary activities that do not adversely affect one another.

Accordingly, in one aspect, the present invention also provides a pharmaceutical combination product. In one embodiment, the combination product comprises the antibody, the immunoconjugate, or the multispecific antibody of the present invention and a second therapeutic agent formulated in the same pharmaceutical composition or formulation. In another embodiment, the combination product comprises the antibody, the immunoconjugate, or the multispecific antibody of the present invention and a second therapeutic agent separately contained in different pharmaceutical compositions or formulations. The second therapeutic agent can be administered prior to, concurrently with (e.g., in the same formulation or in different formulations), or subsequent to the administration of the antibody of the present invention.

The pharmaceutical composition, formulation, and combination product of the present invention can be provided in an article of manufacture for the treatment, prevention, and/or diagnosis of the disease and/or disorder described herein. The article of manufacture can comprise a container and a label or a package insert. Suitable containers include, for example, a bottle, a syringe, an IV infusion bag, and the like. The container may be made of a variety of materials such as glass or plastic. In one embodiment, the article of manufacture can comprise (a) a first container containing the antibody or the antibody fragment, the immunoconjugate, or the multispecific antibody of the present invention; and optionally (b) a second container containing a second therapeutic agent. In addition, the article of manufacture can further comprise other materials desirable from a commercial and user standpoint, including buffers, pharmaceutically acceptable diluents such as sterile water for injection, needles, syringes, syringe pumps, and the like.

## VIII. Applications

In one aspect, the present invention provides a method for preventing and/or treating a B7-H3-related disease or disorder (e.g., cancer), which comprises administering to a subject an effective amount of the anti-B7-H3 antibody or the antigen-binding fragment thereof, the immunoconjugate, or the pharmaceutical composition of the present invention.

The subject may be a mammal, e.g., a primate, preferably a higher primate, such as a human. In one embodiment, the subject suffers from or is at risk of suffering from the disease described herein. In certain embodiments, the subject is receiving or has received additional therapies, e.g., chemotherapy and/or radiotherapy. In other aspects, the present invention provides use of the anti-B7-H3 antibody or the antigen-binding fragment thereof, the immunoconjugate, or the pharmaceutical composition in the manufacture or preparation of a medicament for the prevention and/or treatment of a B7-H3-related disease or disorder mentioned herein.

In some embodiments, the antibody or the antigen-binding fragment thereof, the immunoconjugate, the composition, or the product of the present invention delays the onset of the disorders and/or symptoms associated with the disorders.

The antibody of the present invention (the pharmaceutical composition or the immunoconjugate comprising the same, and any additional therapeutic agents) can be administered by any suitable method, including parenteral administration, intrapulmonary administration, intranasal administration, and, if required by locoregional treatment, intralesional administration. Parenteral infusion includes intramuscular, intravenous, intra-arterial, intraperitoneal or subcutaneous administration. The administration is carried out by any suitable means, such as injection, e.g., intravenous or subcutaneous injection, to some extent depending on whether the treatment is short-term or long-term. Various administration schedules are encompassed herein, including, but not limited to, single administration or multiple administrations at multiple time points, bolus injection, and pulse infusion.

In order to prevent or treat diseases, the appropriate dosage of the antibody of the present invention (when used alone or in combination with one or more other therapeutic agents) will depend on types of diseases to be treated, types of antibodies, severity and progression of the disease, purpose of administration (prophylactic or therapeutic) of the antibody, previous treatments, clinical histories of patients, responses to the antibody, and the discretion of an attending physician. The antibody is suitably administered to a patient through a single treatment or through a series of treatments.

In the methods described above, the composition, multispecific antibody, or immunoconjugate of the present invention can be administered in place of the antibody or the antigen-binding portion thereof of the present invention. Alternatively, in the methods, the composition, multispecific antibody, or immunoconjugate of the present invention can be further administered after the antibody or the antigen-binding portion thereof of the present invention is administered.

**IX. Methods and compositions for diagnosis and detection**

In certain embodiments, any of the anti-B7-H3 antibodies or the antigen-binding fragments thereof provided herein can be used to detect the presence of B7-H3 in a biological sample. In one embodiment, the detection results are used for diagnosis or auxiliary diagnosis of diseases. The term "detection" or "detect" used herein includes quantitative and qualitative detections, and exemplary detections may involve immunohistochemistry, immunocytochemistry, flow cytometry (e.g., FACS), magnetic beads complexed with antibody molecules, ELISA, and PCR (e.g., RT-PCR). In certain embodiments, the biological sample is blood, serum, or other liquid samples of biological origin. In certain embodiments, the biological sample includes cells or tissues. In some embodiments, the biological sample is derived from a hyperproliferative or cancerous lesion.

In one embodiment, the present invention provides a method and a kit for detecting B7-H3 in a biological sample. In certain embodiments, the B7-H3 is human B7-H3 or cynomolgus monkey B7-H3. In certain embodiments, the method comprises contacting the biological sample with the anti-B7-H3 antibody as described herein under conditions allowing binding of the antibody to B7-H3, and detecting whether a complex is formed by the anti-B7-H3 antibody and B7-H3. The method may be an *in vitro* or *in vivo* method. In some embodiments, the sample is from a cancer patient. The sample may be a tissue biopsy, a tissue section, a body fluid such as blood, plasma, or serum.

In some embodiments, provided is a method for treating a B7-H3-related disease or disorder (e.g., cancer or tumor), which comprises: administering to a subject a therapeutically effective amount of the anti-B7-H3 antibody. In another embodiment, the method further comprises administering to the subject one or more other therapies.

In one embodiment, the anti-B7-H3 antibody is used to select a subject eligible for treatment with the anti-B7-H3 antibody, e.g., wherein B7-H3 is a biomarker for selecting the subject. In one embodiment, the antibody of the present invention can be used to diagnose a tumor, e.g., to assess (e.g., monitor) the treatment or progression, diagnosis and/or stage of the disease described herein in a subject. In one embodiment, the presence of cancer cells in various tissues (e.g., ovary, lung, breast, prostate, kidney, pancreas, thyroid, brain, etc.) is determined using the anti-B7-H3 antibody of the present application. The anti-B7-H3 antibody of the present application can be used to determine the presence of cancer cells and the level of B7-H3 released from the solid tumors into the circulating blood, and the B7-H3 antigen in the circulation can be an intact B7-H3 molecule or a fragment thereof. The detection is performed, for example, by FACS method.

In certain embodiments, provided is a labeled anti-B7-H3 antibody. The label includes, but is not limited to, a label or moiety that is detected directly, e.g., a fluorescent label, a chromophoric label, an electron-dense label, a chemiluminescent label, and a radioactive label, and a moiety that is detected indirectly, such as an enzyme or a ligand, for example, by enzymatic reaction or molecular interaction.

**X. Exemplary anti-B7-H3 antibodies of the present invention**

Table A. Amino acid sequences of CDRs of exemplary antibodies of the present invention

| Name of antibody | VH CDR1 (AbM) | VH CDR2 (Kabat) | VH CDR3 (Kabat) | VL CDR1 (Kabat) | VL CDR2 (Kabat) | VL CDR3 (Kabat) |
|---|---|---|---|---|---|---|
| 20G5/ ch20G5 | GYTFTEYIM H (SEQ ID NO:1) | GINPNNGGTTYNQKFK D (SEQ ID NO: 2) | RTPPWHFAV (SEQ ID NO: 3) | SASSSVSYIH (SEQ ID NO: 4) | DTSRLAS (SEQ ID NO: 5) | QQWSSAPL T (SEQ ID NO:6) |
| Hz20G5 | | GINPGTGGTTYNQKFK D (SEQ ID NO: 7) | | | | |
| 19A2/ ch19A2 | GYIFTSYWIH (SEQ ID NO: 8) | RIYPGTDSTFYNEKFK G (SEQ ID NO:9) | ITASDWYFD V (SEQ ID NO: 10) | SVSSSVNSNYL Y (SEQ ID NO:11) | GTSNLAS (SEQ ID NO: 12) | YQWSSYPF T (SEQ ID NO:13) |
| Hz 19A2 | | RIYPGTESTFYNEKFKG (SEQ ID NO: 14) | | SVSSSVQSNYL Y (SEQ ID NO: 15) | | |

EP 4 269 435 A1

Table B. Heavy chain variable regions (VHs) and light chain variable regions (VLs) of exemplary antibodies of the present invention

| Name of antibody | VH Protein | VL Protein |
|---|---|---|
| 19A2 / ch19A2 | EVQLQQSGAELVRPGASVKLSCKTSGYIF TSYWIHWIKQRSGQGLEWIARIYPGTDST FYNEKFKGRATLTADKSSSTVYLQLNSLK SEDSAVYFCHFITASDWYFDVWGAGTTV TVSS (SEQ ID NO: 16) | DIVLTQSPAIMSASPGEKVTLTCSVSSSVNSNYL YWYQQKPGSSPKLWIYGTSNLASGVPARFSGS GSGPSYSLTISSMEAEDAASYFCYQWSSYPFTF GSGTKLEIK (SEQ ID NO: 17) |
| 20G5/ ch20G5 | EVQLQQSVPELVKPGASVKISCKTSGYTF TEYIMHWVKQSHGKNLEWIGGINPNNG GTTYNQKFKDKATLTVDKSSSTAYMELH NLTSEDSAVYYCTRRTPPWHFAVWGAGT SLTVSS (SEQ ID NO: 18) | DIVLTQSPTIMSASPGEKVTMTCSASSSVSYIHW YQQKSGTSPKRWIFDTSRLASGVPARFSGSGSG TSYSLTISSMEAEDAATYYCQQWSSAPLTFGTG TTLELK (SEQ ID NO: 19) |
| Hz19A2 | QVQLVQSGAEVKKPGASVKVSCKASGYI FTSYWIHWVRQAPGQGLEWMGRIYPGT ESTFYNEKFKGRVTMTRDTSTSTVYMEL SSLRSEDTAVYYCHFITASDWYFDVWGQ GTLVTVSS (SEQ ID NO: 20) | EIVLTQSPATLSLSPGERATLSCSVSSSVQSNYLY WYQQKPGQAPRLLIYGTSNLASGIPARFSGSGS GTDFTLTISSLEPEDFAVYYCYQWSSYPFTFGQG TKLEIK (SEQ ID NO: 21) |
| Hz20G5 | QVQLVQSGAEVKKPGASVKVSCKASGY TFTEYIMHWVRQAPGQRLEWMGGINPG TGGTTYNQKFKDRVTITVDTSASTAYME LSSLRSEDTAVYYCTRRTPPWHFAVWGQ GTLVTVSS (SEQ ID NO: 22) | DIQLTQSPSFLSASVGDRVTITCSASSSVSYIHW YQQKPGKAPKRWIYDTSRLASGVPSRFSGSGSG TEFTLTISSLQPEDFATYYCQQWSSAPLTFGGGT KVEIK (SEQ ID NO: 23) |

Table C. Some exemplary sequences of the present invention

| Name of sequence | Sequence |
|---|---|
| Hz19A2-HC | QVQLVQSGAEVKKPGASVKVSCKASGYIFTSYWIHWVRQAPGQGLEWMGRIYPGTESTFYN EKFKGRVTMTRDTSTSTVYMELSSLRSEDTAVYYCHFITASDWYFDVWGQGTLVTVSSASTK GPSVFPLAPSSKSTSGGTAALGCLVKDYFPEPVTVSWNSGALTSGVHTFPAVLQSSGLYSLSSV VTVPSSSLGTQTYICNVNHKPSNTKVDKKVEPKSCDKTHTCPPCPAPELLGGPSVFLFPPKPKD TLMISRTPEVTCVVVDVSHEDPEVKFNWYVDGVEVHNAKTKPREEQYNSTYRVVSVLTVLH QDWLNGKEYKCKVSNKALPAPIEKTISKAKGQPREPQVYTLPPSRDELTKNQVSLTCLVKGFY PSDIAVEWESNGQPENNYKTTPPVLDSDGSFFLYSKLTVDKSRWQQGNVFSCSVMHEALHNH YTQKSLSLSPGK (SEQ ID NO: 24) |
| Hz19A2-LC | EIVLTQSPATLSLSPGERATLSCSVSSSVQSNYLYWYQQKPGQAPRLLIYGTSNLASGIPARFSG SGSGTDFTLTISSLEPEDFAVYYCYQWSSYPFTFGQGTKLEIKRTVAAPSVFIFPPSDEQLKSGTA SVVCLLNNFYPREAKVQWKVDNALQSGNSQESVTEQDSKDSTYSLSSTLTLSKADYEKHKV YACEVTHQGLSSPVTKSFNRGEC (SEQ ID NO: 25) |
| Hz20G5-HC | QVQLVQSGAEVKKPGASVKVSCKASGYTFTEYIMHWVRQAPGQRLEWMGGINPGTGGTTY NQKFKDRVTITVDTSASTAYMELSSLRSEDTAVYYCTRRTPPWHFAVWGQGTLVTVSSASTKG PSVFPLAPSSKSTSGGTAALGCLVKDYFPEPVTVSWNSGALTSGVHTFPAVLQSSGLYSLSSVV TVPSSSLGTQTYICNVNHKPSNTKVDKKVEPKSCDKTHTCPPCPAPELLGGPSVFLFPPKPKDT LMISRTPEVTCVVVDVSHEDPEVKFNWYVDGVEVHNAKTKPREEQYNSTYRVVSVLTVLHQ DWLNGKEYKCKVSNKALPAPIEKTISKAKGQPREPQVYTLPPSRDELTKNQVSLTCLVKGFYP SDIAVEWESNGQPENNYKTTPPVLDSDGSFFLYSKLTVDKSRWQQGNVFSCSVMHEALHNHY TQKSLSLSPGK (SEQ ID NO: 26) |
| Hz20G5-LC | DIQLTQSPSFLSASVGDRVTITCSASSSVSYIHWYQQKPGKAPKRWIYDTSRLASGVPSRFSGS GSGTEFTLTISSLQPEDFATYYCQQWSSAPLTFGGGTKVEIKRTVAAPSVFIFPPSDEQLKSGTAS VVCLLNNFYPREAKVQWKVDNALQSGNSQESVTEQDSKDSTYSLSSTLTLSKADYEKHKVY ACEVTHQGLSSPVTKSFNRGEC (SEQ ID NO: 27) |

| Name of sequence | Sequence |
|---|---|
| 19A2-HC / ch19A2-HC | EVQLQQSGAELVRPGASVKLSCKTSGYIFTSYWIHWIKQRSGQGLEWIARIYPGTDSTFYNEK FKGRATLTADKSSSTVYLQLNSLKSEDSAVYFCHFITASDWYFDVWGAGTTVTVSSASTKGPS VFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSCDKTHTCPPCPAPELLGGPSVFLFPPKPKDTLM ISRTPEVTCVVVDVSHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQVSLTCLVKGFYPSDI AVEWESNGQPENNYKTTPPVLDSDGSFFLYSKLTVDKSRWQQGNVFSCSVMHEALHNHYTQ KSLSLSPGK (SEQ ID NO: 28) |
| 19A2-LC / ch19A2-LC | DIVLTQSPAIMSASPGEKVTLTCSVSSSVNSNYLYWYQQKPGSSPKLWIYGTSNLASGVPARFS GSGSGPSYSLTISSMEAEDAASYFCYQWSSYPFTFGSGTKLEIKRTVAAPSVFIFPPSDEQLKSG TASVVCLLNNFYPREAKVQWKVDNALQSGNSQESVTEQDSKDSTYSLSSTLTLSKADYEKHK VYACEVTHQGLSSPVTKSFNRGEC(SEQ ID NO: 29) |
| 20G5-HC / ch20G5-HC | EVQLQQSVPELVKPGASVKISCKTSGYTFTEYIMHWVKQSHGKNLEWIGGINPNNGGTTYNQ KFKDKATLTVDKSSSTAYMELHNLTSEDSAVYYCTRRTPPWHFAVWGAGTSLTVSSASTKGPS VFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSCDKTHTCPPCPAPELLGGPSVFLFPPKPKDTLM ISRTPEVTCVVVDVSHEDPEVKFNWYVDGVEVHNAKTKPREEQYNSTYRVVSVLTVLHQDW LNGKEYKCKVSNKALPAPIEKTISKAKGQPREPQVYTLPPSRDELTKNQVSLTCLVKGFYPSDI AVEWESNGQPENNYKTTPPVLDSDGSFFLYSKLTVDKSRWQQGNVFSCSVMHEALHNHYTQ KSLSLSPGK (SEQ ID NO: 30) |
| 20G5-LC / ch20G5-LC | DIVLTQSPTIMSASPGEKVTMTCSASSSVSYIHWYQQKSGTSPKRWIFDTSRLASGVPARFSGS GSGTSYSLTISSMEAEDAATYYCQQWSSAPLTFGTGTTLELKRTVAAPSVFIFPPSDEQLKSGTA SVVCLLNNFYPREAKVQWKVDNALQSGNSQESVTEQDSKDSTYSLSSTLTLSKADYEKHKV YACEVTHQGLSSPVTKSFNRGEC (SEQ ID NO: 31) |

EP 4 269 435 A1

| Name of sequence | Sequence |
|---|---|
| Human 4Ig-B7H3 | MLRRRGSPGMGVHVGAALGALWFCLTGALEVQVPEDPVVALVGTDATLCCSFSPEPGFSLAQ LNLIWQLTDTKQLVHSFAEGQDQGSAYANRTALFPDLLAQGNASLRLQRVRVADEGSFTCFVS IRDFGSAAVSLQVAAPYSKPSMTLEPNKDLRPGDTVTITCSSYQGYPEAEVFWQDGQGVPLTG NVTTSQMANEQGLFDVHSILRVVLGANGTYSCLVRNPVLQQDAHSSVTITPQRSPTGAVEVQ VPEDPVVALVGTDATLRCSFSPEPGFSLAQLNLIWQLTDTKQLVHSFTEGRDQGSAYANRTALF PDLLAQGNASLRLQRVRVADEGSFTCFVSIRDFGSAAVSLQVAAPYSKPSMTLEPNKDLRPGD TVTITCSSYRGYPEAEVFWQDGQGVPLTGNVTTSQMANEQGLFDVHSVLRVVLGANGTYSC LVRNPVLQQDAHGSVTITGQPMTFPPEALWVTVGLSVCLIALLVALAFVCWRKIKQSCEEENA GAEDQDGEGEGSKTALQPLKHSDSKEDDGQEIA (SEQ ID NO: 32) |
| Human 2Ig-B7H3 | MLRRRGSPGMGVHVGAALGALWFCLTGALEVQVPEDPVVALVGTDATLCCSFSPEPGFSLAQ LNLIWQLTDTKQLVHSFAEGQDQGSAYANRTALFPDLLAQGNASLRLQRVRVADEGSFTCFVS IRDFGSAAVSLQVAAPYSKPSMTLEPNKDLRPGDTVTITCSSYRGYPEAEVFWQDGQGVPLTG NVTTSQMANEQGLFDVHSVLRVVLGANGTYSCLVRNPVLQQDAHGSVTITGQPMTFPPEAL WVTVGLSVCLIALLVALAFVCWRKIKQSCEEENAGAEDQDGEGEGSKTALQPLKHSDSKEDD GQEIA (SEQ ID NO: 33) |
| Cynomolgus monkey B7H3 | MLHRRGSPGMGVHVGAALGALWFCLTGALEVQVPEDPVVALVGTDATLRCSFSPEPGFSLAQ LNLIWQLTDTKQLVHSFTEGRDQGSAYANRTALFLDLLAQGNASLRLQRVRVADEGSFTCFVS IRDFGSAAVSLQVAAPYSKPSMTLEPNKDLRPGDTVTITCSSYRGYPEAEVFWQDGQGAPLTG NVTTSQMANEQGLFDVHSVLRVVLGANGTYSCLVRNPVLQQDAHGSITITPQRSPTGAVEVQ VPEDPVVALVGTDATLRCSFSPEPGFSLAQLNLIWQLTDTKQLVHSFTEGRDQGSAYANRTALF LDLLAQGNASLRLQRVRVADEGSFTCFVSIRDFGSAAVSLQVAAPYSKPSMTLEPNKDLRPGD TVTITCSSYRGYPEAEVFWQDGQGAPLTGNVTTSQMANEQGLFDVHSVLRVVLGANGTYSC LVRNPVLQQDAHGSVTITGQPMTFPPEALWVTVGLSVCLVALLVALAFVCWRKIKQSCEEEN AGAEDQDGEGEGSKTALQPLKHSDSKEDDGQELA (SEQ ID NO: 34) |

| Name of sequence | Sequence |
|---|---|
| MGA271 VH | EVQLVESGGGLVQPGGSLRLSCAASGFTFSSFGMHWVRQAPGKGLEWVAYISSDSSAIYYADT VKGRFTISRDNAKNSLYLQMNSLRDEDTAVYYCGRGRENIYYGSRLDYWGQGTTVTVSS (SEQ ID NO: 35) |
| MGA271 VL | DIQLTQSPSFLSASVGDRVTITCKASQNVDTNVAWYQQKPGKAPKALIYSASYRYSGVPSRFS GSGSGTDFTLTISSLQPEDFATYYCQQYNNYPFTFGQGTKLEIK (SEQ ID NO: 36) |
| Human IgG1-CH | ASTKGPSVFPLAPSSKSTSGGTAALGCLVKDYFPEPVTVSWNSGALTSGVHTFPAVLQSSGLYS LSSVVTVPSSSLGTQTYICNVNHKPSNTKVDKKVEPKSCDKTHTCPPCPAPELLGGPSVFLFPP KPKDTLMISRTPEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPSRDELTKNQVSLTCLV KGFYPSDIAVEWESNGQPENNYKTTPPVLDSDGSFFLYSKLTVDKSRWQQGNVFSCSVMHEA LHNHYTQKSLSLSPGK (SEQ ID NO: 37) |
| Human κ-CL | RTVAAPSVFIFPPSDEQLKSGTASVVCLLNNFYPREAKVQWKVDNALQSGNSQESVTEQDSK DSTYSLSSTLTLSKADYEKHKVYACEVTHQGLSSPVTKSFNRGEC (SEQ ID NO: 38) |

**Examples**

The following examples are described to assist in understanding the present invention. The examples are not intended to, and should not be construed as, limiting the protection scope of the present invention in any way, and various modifications may be made by those skilled in the art according to the description of the specification of the present application.

Unless otherwise indicated, conventional methods of chemistry, biochemistry, organic chemistry, molecular biology, microbiology, recombinant DNA techniques, genetics, immunology and cell biology that are known in the art will be employed for the implementation of the present invention. Descriptions of such methods can be found, for example, in Sambrook et al., Molecular Cloning: A Laboratory Manual (3rd Ed., 2001); Sambrook et al., Molecular Cloning: A Laboratory Manual (2nd Ed., 1989); Maniatis et al., Molecular Cloning: A Laboratory Manual (1982); Ausubel et al., Current Protocols in Molecular Biology (John Wiley and Sons, updated in July 2008); Short Protocols in Molecular Biology: A Compendium of Methods from Current Protocols in Molecular Biology, Greene Pub. Associates and Wiley-Interscience; Glover, DNA Cloning: A Practical Approach, vol.I&II (IRL Press, Oxford, 1985); Anand, Techniques for the Analysis of Complex Genomes, (Academic Press, New York, 1992); Transcription and Translation (B. Hames&S. Higgins, Eds., 1984); Perbal, A Practical Guide to Molecular Cloning (1984); Harlow and Lane, Antibodies (Cold Spring Harbor Laboratory Press, Cold Spring Harbor, N.Y., 1998); Current Protocols in Immunology (Q. E. Coligan, A. M. Kruisbeek, D. H. Margufies, E. M. Shevach and W. Strober, eds., 1991); Annual Review of Immunology, and journals and monographs such as Advances in Immunology.

**Example 1. Preparation of Hybridoma Cells**

Animal immunization

Bal b/c mice (Beijing Vital River Laboratory Animal Technology Co., Ltd.) were immunized with recombinant human 4Ig-B7H3 protein (SEQ ID NO: 32)(SINO BIOLOGICAL, Catalog No. 11188-H08H) according to a conventional method. The recombinant human 4Ig-B7H3 protein (50 $\mu$g per mouse) was mixed well with an equal volume of TiterMax (Sigma, Catalog No. T2684-1ML) adjuvant. The mixture was then injected subcutaneously once every two weeks for a total of 5 immunizations.

Cell fusion

When the serum titer met the requirement, according to a conventional method, the spleen of a mouse was collected to prepare a B lymphocyte suspension. The B lymphocyte suspension was then mixed with SP2/0 myeloma cells (ATCC, CRL-1581) in a ratio of 1:2-1:1 to perform electrofusion. The fused cells were transferred from an electrode dish to a 50 mL centrifuge tube and diluted with a screening medium (the preparation composition is shown in Table 1) to obtain a cell suspension (with a concentration of 1-2 $\times$ $10^4$ cells/mL). 100 $\mu$L of cell suspension was added to each well of a 96-well plate. The fresh screening medium was refreshed 5 days after the fusion. Positive clones were screened by flow cytometry (FACS) detection after 10 days (or longer) of culture according to the growth state of the cells.

Table 1: Screening medium

| Name | Composition | Preparation |
|---|---|---|
| Screening medium | RPMI-1640 (Hyclone) | 90% |
| | FBS (Hyclone) | 10% |
| | HAT medium (Gibco) | 1$\times$ |
| | GlutaMAX™ Supplement (Gibco) | 1$\times$ |

High-throughput screening of hybridoma cells

Hybridoma cells specifically expressing the anti-B7H3 antibody were screened out by a flow cytometer (FACS). Briefly, CHO cells expressing human B7H3 (CHO-huB7H3) were counted, diluted to 1 $\times$ $10^6$ cells/mL, and added to a U-bottom 96-well plate at 100 $\mu$L/well. The cell suspension was centrifuged at 500 g for 5 min to remove the cell medium. Then, the culture supernatant and positive control antibody (MGA271) in the above hybridoma 96-well plate were added to a U-shaped plate containing CHO cells at 100 $\mu$L/well, and the cells were resuspended, and the suspension was left to stand on ice for 30 min. The mixture was centrifuged at 500 g for 5 min to remove the supernatant, and the cells were washed once with a PBS solution. The mixture was centrifuged at 500 g for 5 min to remove the PBS solution. 100 $\mu$L

of FITC-labeled anti-mouse Fab secondary antibody (1:500 dilution in PBS solution) was added to each well, and 100 μL of FITC-labeled anti-human Fab secondary antibody was added to the positive control antibody culture cell. The mixture was incubated in the dark on ice for 30 min, and then centrifuged at 500 g for 5 min to remove the supernatant. The cells were washed once with a PBS solution. The cells were then resuspended with 50 μL of PBS solution, FACS detection was performed, and positive clones were obtained by screening.

The obtained positive clones were rescreened using CHO cells (CHO-cynoB7H3) expressing cynomolgus monkey B7H3 (SEQ ID NO: 34) by the same method described above to obtain 2 strains of hybridoma cells binding to both human B7H3 and monkey B7H3: 19A2 and 20G5.

The affinity of the obtained 2 strains of hybridoma cells for the antigen was determined by using bio-layer interferometry (ForteBio). The obtained KD values are shown in Table 2.

Table 2: ForteBio affinity determination results

| Hybridoma clone No. | Human 4Ig-B7H3 | Cynomolgus monkey B7H3 |
|---|---|---|
| 19A2 | 9.87E-10 | 9.77E-10 |
| 20G5 | 3.62E-10 | 5.90E-10 |

Subcloning of positive hybridoma cell

The above clones were subcloned according to the results of cell binding and affinity assays.

The specific steps were as follows: HAT in the screening medium was changed to HT (Gibco, Cat# 11067-030) to obtain a basal medium, and the medium was added to a 96-well plate at 200 μL/well. 300 μL of positive hybridoma cells screened by the fusion were added to each well in the first row of the 96-well plate at a density of about $1 \times 10^5$ cells/mL, and the suspension was well mixed. 100 μL of the cell suspension in the first row was transferred into the second row, and 100 μL of the mixture was transferred into the next row after mixing well. The above steps were repeated until the mixture was transferred into the last row, and the mixture was left to stand for 15 min. The cells were counted under a microscope. A corresponding volume containing 100 cells was added to 20 mL of the basal medium described above for mixing and plating at 200 μL/well. After two days, the cells were observed under a microscope, and monoclonal wells were identified and marked. When the cell confluence in each well reached more than 50%, the high-throughput FACS screening method was used for detection, target positive wells were picked out, and the obtained cell clones were frozen.

The positive control antibody used in the present invention was MGA271, also referred to as Enoblituzumab (from MacroGenics US20160264672A1).

**Example 2. Preparation of Chimeric Antibody**

Antibody light and heavy chain gene sequences of the hybridoma positive clones obtained in Example 1 were extracted by using molecular biology techniques and were used to construct a human-mouse chimeric antibody.

1. Hybridoma sequencing

RNA was extracted from about $5 \times 10^6$ hybridoma cells freshly cultured, and reverse transcription was performed by using PrimeScript II 1st Strand cDNA Synthesis Kit (Takara) to obtain cDNA. The steps were as follows:
The reaction system I in Table 3 was prepared.

Table 3:

| Name | Amount |
|---|---|
| Oligo dT primer | 1 μL |
| dNTP | 1 μL |
| Template RNA (RNA obtained above) | 5 ug |
| RNase-free ddH$_2$O | Make up to 10 μL |

After incubation at 65 °C for 5 min, the system was rapidly cooled on ice. The reaction system I was added to the following reverse transcription system (Table 4) in a total amount of 20 μL.

Table 4: Reverse transcription system

| Name | Amount |
|---|---|
| Reaction system I | 10 μL |
| 5 × PrimeScript II Buffer | 4 μL |
| RNase inhibitor (40 U/μL) | 0.5 μL (20 U) |
| PrimeScript II RTase (200U/ μL) | 1 μL (200U) |
| RNase-free ddH$_2$O | Make up to 20 μL |

After slowly mixing, reverse transcription was induced in the following conditions of 42 °C for 60 min → 95 °C for 5 min. The mixture was then cooled on ice before cDNA collection.

The cDNA was ligated to a T vector, and then the heavy and light chain variable regions of the antibody were each amplified from the obtained cDNA by PCR using Mighty TA-cloning Kit (Takara). The PCR reaction system is shown in Table 5.

Table 5:

| Name | Amount |
|---|---|
| TaKaRa EX Tag HS | 0.25 μL, |
| Primer Mix 1 (Table 7) | 1 μL |
| Primer Mix 2 (Table 8) | 1 μL |
| cDNA | 1 μL |
| 10 × Ex Tag buffer | 5 μL, |
| dNTP Mixture (2.5 mM each) | 4 μL |
| RNase-free ddH$_2$O | Make up to 50 μL |

The PCR reaction conditions are shown in Table 6.

Table 6:

| 94 °C | 5 min | |
| 94 °C | 30 s | 30 cycles |
| 55 °C | 30 s | |
| 72 °C | 60 s | |
| 72 °C | 5 min | |

0.5 μL of pMD20-T vector (Takara) and 5 μL of Ligation Mighty Mix (Takara) were added to 4.5 μL of the PCR product from the above PCR reaction. The mixture was mixed well gently, and incubated at 37 °C for 2 h to obtain a ligation product.

Transformation of cells:

5 μL of the obtained ligation product was added to *E. coli* TOP10 competent cells (Tiangen Biotech (Beijing) Co., Ltd.). The mixture was mixed well and then incubated on ice for 30 min. After heat shock at 42 °C for 90 s, the obtained mixture was rapidly cooled on ice for 2 min. An additional 900 μL of LB culture medium (Sangon Biotech (Shanghai) Co., Ltd.) was added to the EP tube, and the mixture was incubated at 37 °C on a shaker at 220 rpm for 1 h. The cells were centrifuged at 3000 g for 2 min. 800 μL of supernatant was discarded, and the cells were resuspended in the remaining medium for coating an ampicillin plate. The cells were incubated overnight at 37 °C. Clones were picked for sequencing.

2. Construction of chimeric antibodies

The VH and VL regions, which had been sequenced, of the anti-B7H3 antibody generated from the hybridoma cells in Example 1 were amplified by PCR: the sequences of forward and reverse primers are shown in Tables 7 and 8.

Table 7. Primer (Primer Mix 1) for heavy chain variable region (VH) of mouse antibody

| Name of primer | Sequence (5'-3') | Ratio (%) |
|---|---|---|
| OVH1 | SAGGTCCAGCTGCAGCAGYYTGG (SEQ ID NO: 39) | 28.6 |
| OVH2 | CAGGTRCAGCTGAAGSAGTCAGG (SEQ ID NO: 40) | 10.7 |
| OVH3 | GAKGTGCAGCTTCAGCAGTCRGG (SEQ ID NO: 41) | 8.9 |
| OVH5 | GAVGTGAWGCTGGTGGAGTCTGR (SEQ ID NO: 42) | 7.1 |
| OVH11 | GAAGTGCAGCTGTTGGAGACTGG (SEQ ID NO: 43) | 3.6 |
| OVH14 | GAGGTTCAGCTGCAGCAGTCTGK (SEQ ID NO: 44) | 16.1 |
| OVH15 | CAGGTTCACCTACAACAGTCTGG (SEQ ID NO: 45) | 3.5 |
| REVESE-6 | CTGAGGARACGGTGACCG (SEQ ID NO: 46) | 6 |
| REVESE-4 | CTGAGGAGACTGTGAGAGWGGT (SEQ ID NO: 47) | 4 |
| REVESE-2-1 | CTGAGGAGACGGTGACTGAGGT (SEQ ID NO: 48) | 2 |
| REVESE-2-2 | CTGCAGAGACAGTGACCAGAGT (SEQ ID NO: 49) | 2 |
| Water | | q.s. |

After components were mixed in the above proportions, the resulting Primer Mix 1 was used for subsequent VH PCR amplification.

Table 8. Primer (Primer Mix 2) for light chain variable region (VL) of mouse antibody

| Name of primer | Sequence (5'-3') | Ratio (%) |
|---|---|---|
| IGKV1 | GATGYTKTGATGACCCAAACTCCA (SEQ ID NO: 50) | 17.65 |
| IGKV2-109 | GATATTGTGATGACGCAGGCTGCA (SEQ ID NO: 51) | 5.88 |
| IGKV2-112 | GATATTGTGATAACCCAGGATGAA (SEQ ID NO: 52) | 5.88 |
| IGKV3-7 | GACATTGTGCTAACACAGTCTCCT (SEQ ID NO: 53) | 2.94 |
| IGKV3-1-5.10 | RACATTGTGCTSACCCAATCTCCA (SEQ ID NO: 54) | 29.41 |
| IGKV5-48 | GACATCTTGCTGACTCAGTCTCCA (SEQ ID NO: 55) | 2.94 |
| IGKV6-13 | GACATTGTGATGACCCAGTCTCAA (SEQ ID NO: 56) | 2.94 |
| IGKV6-32 | AGTATTGTGATGACCCAGACTCCC (SEQ ID NO: 57) | 2.94 |
| IGKV14 | GACATCMAGATGACMCAGTCTCCA (SEQ ID NO: 58) | 11.76 |
| IGKV4-51.86 | GAAAATGTGCTCACYCAGTCTCCA (SEQ ID NO: 59) | 2.94 |
| IGKV7-33 | GACATTGTGATGACTCAGTCTCCA (SEQ ID NO: 60) | 2.94 |
| IGKV9-123 | GACATCCAGATGATTCAGTCTCCA (SEQ ID NO: 61) | 2.94 |
| IGKV9-124 | GACATCCAGATGACCCAGTCTCCA (SEQ ID NO: 62) | 2.94 |
| IGKV10-95 | GATATCCAGATGACACAGACTACT (SEQ ID NO: 63) | 2.94 |
| IGKV11-125 | GATGTCCAGATGATTCAGTCTCCA (SEQ ID NO: 64) | 2.94 |
| mK-Rev | TACAGTTGGTGCAGCATCAG (SEQ ID NO: 65) | |

After components were mixed in proportions, the resulting Primer Mix 2 was used for subsequent VL PCR amplification.

The PCR system is shown in Table 9.

Table 9:

| Name | Amount |
|---|---|
| 2×Prime STAR HS (Premix) | 25 μL |
| Primer Mix* | 2 μL |
| Plasmid template | 0.5 μL |

(continued)

| Name | Amount |
|---|---|
| dNTP Mixture (2.5 mM each) | 4 μL |
| RNase free ddH$_2$O | Make up to 50 μL |

* For VH amplification, Primer Mix 1 was used; for VL amplification, Primer Mix 2 was used. The gel was cut to recover the PCR amplification products.

Homologous recombination:

The homologous recombination system is shown in Table 10.

Table 10:

| Name | Amount |
|---|---|
| Recovering fragments | 1 μL |
| pTT5 vector | 2 μL |
| 5 × Buffer (Takara) | 2 μL |
| Homologous recombination enzyme (Takara) | 1 μL |
| ddH$_2$O | Make up to 10 μL |

After incubation at 37 °C for 30 min, a recombinant product was obtained. The TOP10 competent cells were transformed by the recombinant product, and monoclones were picked for sequencing. Clones containing plasmids with correct insertion directions were selected as positive clones and preserved, thus obtaining the recombinant plasmids of the chimeric antibody. A certain amount of recombinant plasmids was prepared and extracted for expressing the antibody.

Two chimeric antibodies (Ch19A2 and Ch20G5) were obtained in the present invention, the CDR sequences and the light and heavy chain variable region sequences were identical to the corresponding sequences of the hybridoma cells in Tables A-B, and the preferred amino acid sequences of the light and heavy chains of the chimeric antibodies are shown in Table C.

3, Expression and purification of chimeric antibodies

HEK293 cells (Invitrogen) were passaged according to a desired transfection volume. The cell density was adjusted to $1.5 \times 10^6$ cells/mL the day before transfection. The cell density on the day of transfection was approximately $3 \times 10^6$ cells/mL. 1/10 (v/v) of the final volume of Opti-MEM medium (Gibco, Catalog No. 31985-070) was taken as a transfection buffer. The recombinant expression plasmids constructed as described above were added. The mixture was mixed well, and filtered with a 0.22 μm filter for later use. An appropriate amount of polyethylenimine (PEI) (Polysciences, 23966) was added to the plasmids from the previous step (the mass ratio of plasmids to PEI was 1:3), mixed well and incubated at room temperature for 10 min to give a DNA/PEI mixture. The DNA/PEI mixture was gently poured into HEK293 cells, mixed well, and cultured at 37 °C, 8% CO$_2$ for 24 h, followed by the addition of VPA (Sigma, Catalog No. P4543-100G) at a final concentration of 2 mM. Then 2% (v/v) Feed solution (1 g/L Phytone Peptone + 1 g/L Difco Select Phytone) was added and the resulting mixture was cultured for another 6 days.

After culture, the cell culture fluid was centrifuged at 13000 rpm for 20 min, the supernatant was collected and purified by a pre-packed column Hitrap Mabselect Sure (GE, 11-0034-95) according to the manufacturer's instructions, and then the concentration was determined. 100 μg of the purified protein was taken with its concentration adjusted to 1 mg/mL. The protein purity was determined using a gel filtration column SW3000 (TOSOH Catalog No. 18675). The results show that a chimeric antibody with high purity was obtained.

**Example 3. Binding Kinetics of Chimeric Antibodies of the Present Invention for Antigens as Determined by Bio-Layer Interferometry**

The equilibrium dissociation constant (KD) for binding of the antibodies of the present invention to human B7H3 was determined by bio-layer interferometry (ForteBio). The ForteBio affinity assay was conducted according to the method (Estep, P et al., High throughput solution Based measurement of antibody-antigen affinity and epitope binding. mAbs, 2013.5(2): 270-8) known in the art.

Briefly, AMQ (Pall, 1506091) (for sample detection) or AHQ (Pall, 1502051) (for positive control detection) sensors were equilibrated offline in an assay buffer for 30 min, and were equilibrated online for 60 s to establish a baseline. The purified antibodies obtained as described above were loaded online onto an AHQ sensor (ForteBio) for the ForteBio affinity assay. The sensor with the loaded antibodies was then exposed to the antigens (including human 4Ig-B7H3, human 2Ig-B7H3 (ACRO, Catalog No. B73-H52E2) and cynomolgus monkey B7H3 (SINO BIOLOGICAL, Catalog No. 90806-C02H-50)) before transferring the sensor to the assay buffer for dissociation rate measurement. The KD values were analyzed using ForteBio analysis software.

The results of the antibody affinity assays are shown in Table 11:

Table 11. Affinity (equilibrium dissociation constant KD) for bindings of antigens and antibodies by ForteBio assay

| Antibody | Human 4Ig-B7H3 | Human 2Ig-B7H3 | Cynomolgus monkey B7H3 |
|---|---|---|---|
| Ch19A2 | 3.62E-10 | 5.74E-09 | 5.90E-10 |
| Ch20G5 | 2.36E-10 | 3.18E-09 | 7.13E-10 |
| MGA271 | 6.36E-10 | 1.87E-07 | 1.66E-09 |

According to the above affinity data, it can be seen that the chimeric antibodies obtained from the hybridoma have good affinity for human B7H3 protein, and also maintain high affinity for cynomolgus monkey B7H3. The antibodies of this study have higher affinity, compared with MGA271 of the control group.

Example 4. Humanization of Chimeric Antibodies

The chimeric antibodies obtained in Example 2 were humanized according to a conventional method. Thus, humanized antibodies (hz20G5 and hz19A2) were obtained. The CDR sequences, the light and heavy chain variable region sequences, and the amino acid sequences of the light chain and heavy chain are shown in Tables A-C.

Example 5. Affinity of Humanized Antibodies for Antigens as Determined by ForteBio

The binding affinity of the humanized antibodies obtained in Example 4 for the antigens (human B7H3 and cynomolgus monkey B7H3) was determined by ForteBio assay as described in Example 3, which was expressed as an equilibrium dissociation constant (KD). The results are shown in Table 12.

Table 12. Affinity constants (M) for bindings of antigens and antibodies by ForteBio assay

| Antibody | Human 4Ig-B7H3 | Human 2Ig-B7H3 | Cynomolgus monkey B7H3 |
|---|---|---|---|
| Hz20G5 | 3.71E-10 | 6.30E-09 | 4.60E-10 |
| Hz19A2 | 6.01E-10 | 1.04E-08 | 9.68E-10 |
| MGA271 | 9.39E-10 | 7.82E-07 | 1.87E-09 |

As can be seen from Table 12, the humanized antibodies still have high affinity for antigen B7H3, and have equilibrium dissociation constants $K_D$ comparable to those of the corresponding chimeric antibodies against antigen B7H3. In addition, the humanized antibodies obtained in the present application have higher antigen-binding affinity than that of the control antibody MGA271, especially for human 2Ig-B7-H3. The affinity of the antibodies of the present application is improved by 20-100 times compared with that of MGA271.

**Example 6. Binding of Humanized Antibodies to CHO-S Cells Overexpressing Human and Cynomolgus Monkey B7H3**

To verify whether the antibody of the present invention can bind to an antigen expressed on the cell surface, the binding of the humanized antibodies of the present application to cells overexpressing human B7H3 and cynomolgus monkey B7H3 was determined by flow cytometry.

Construction of cells overexpressing B7H3

Using the ExpiCHO™ Expression System Kit (Invitrogen, Catalog No. A29133), the following operations were carried out according to the manufacturer's instructions: cDNAs encoding human 4Ig-B7H3 (uniprot: Q5ZPR3, SEQ ID NO: 32), human 2Ig-B7H3 (uniprot: Q5ZPR3-2, SEQ ID NO: 33) and cynomolgus monkey B7H3 (NCBI: XP_015308534.1, SEQ ID NO: 34) were cloned into the pCHO1.0 vector (Invitrogen), followed by transfection of CHO-S cells to generate CHO-S cells overexpressing human 4Ig-B7H3, human 2Ig-B7H3 and cynomolgus monkey B7H3: CHOS-hB7H3-4Ig, CHOS-hB7H3-2Ig, and CHOS-cyno B7H3.

Briefly:

1) CHOS-hB7H3-4Ig, CHOS-hB7H3-2Ig, and CHOS-cyno B7H3 cells were diluted with a PBS solution to $2 \times 10^6$ cells/mL, and added to a U-bottom 96-well plate at 100 μL/well. Three-fold serially diluted antibodies were added.
2) The above mixture was incubated on ice for 30 min. The cell suspension was centrifuged at 400 g for 5 min to remove the supernatant, and the cells were washed with a PBS solution to remove the unbound antibodies. 100 μL of the solution of PE-conjugated anti-human Fc antibody (SouthernBiotech) diluted at a ratio of 1:200 was added to each well, and the cells were incubated in the dark on ice for 30 min. Then the cell suspension was centrifuged at 400 g for 5 min to remove the supernatant. The cells were washed with PBS twice to remove the unbound PE-conjugated anti-human Fc antibody. Then the cells were resuspended with 100 μL of PBS, and the binding of the antibody to cells was assayed by FACS.

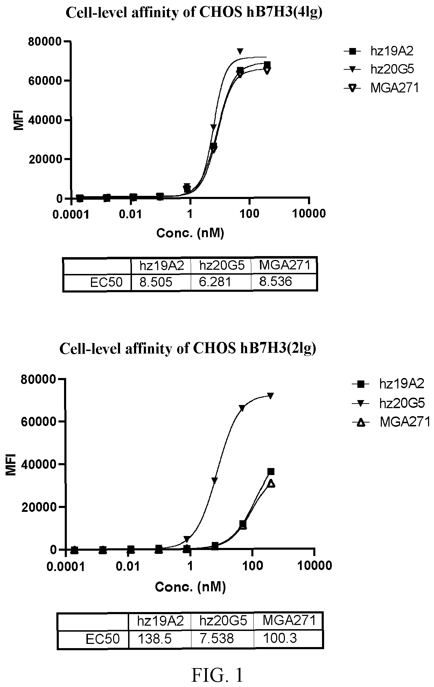

As shown in FIG. 1, the overall affinity of the humanized antibodies of the present application was comparable to that of the positive control MGA271 with respect to cells overexpressing human 4Ig-B7H3. The affinity of the hz19A2 antibody was comparable to that of MGA271, while the affinity of hz20G5 was significantly higher than that of MGA271 with respect to cells overexpressing human 2Ig-B7H3. It can be seen that the antibodies of the present application exhibit significantly improved antigen-binding capacity at the cellular level.

## Example 7. Antibody-Dependent Cell-Mediated Cytotoxicity (ADCC)

This example investigates the effect of the obtained antibodies in mediating an ADCC effect and thereby eliminating tumor cells. In this study, the Jurkat-ADCCNF-AT luciferase effector cell line (hereinafter referred to as ADCC effector cells) from Promega was used. The ADCC activity of the antibodies was detected by detecting the activation of NF-AT signal. The specific experimental process is as follows:

1) Preparation of cells
CHO-hB7H3-4Ig cells and ADCC effector cells were counted. The supernatant was removed by centrifugation. The cells were washed twice with PBS and resuspended in a detection medium (1640 medium with 5% low IgG serum (Gibco)). The concentration of ADCC effector cells was adjusted to $1 \times 10^7$ cells/mL and the concentration of CHO-hB7H3-4Ig cells was adjusted to $1 \times 10^6$ cells/mL. The two types of cells were mixed at 1:1, and the final ratio of ADCC effector cells to CHO-hB7H3-4Ig cells was 10:1.
2) Plating: the mixed cells were plated onto a 96-well plate at 100 μL/well, and an additional 50 μL of cells were added to the first well.
3) The antibodies of the present invention with different concentration gradients were added in sequence: the final concentration of the initial well was 30 nM, followed by three-fold dilution for 10 gradients in total.
4) The cells were incubated in an incubator at 37 °C for 7 h.
5) After 7 h, the 96-well plate was taken out and 100 μL of thawed Luciferase test reagent was added to each well. The cells were incubated at room temperature for 20 min, and detected using a microplate reader. The concentration-dependent curve was fitted with GraphPad software.

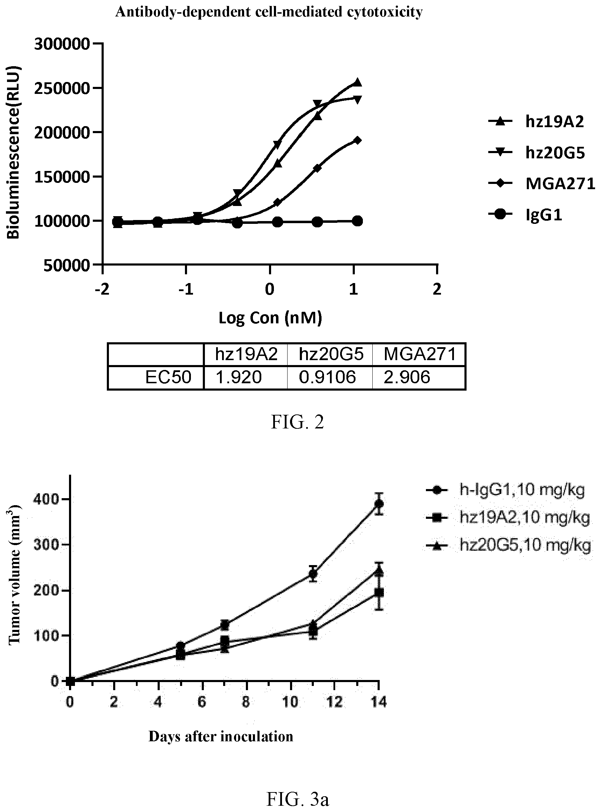

As shown in FIG. 2, both the humanized antibodies and the chimeric antibodies obtained in the present application can effectively activate NF-AT signal, which is a downstream signaling pathway of ADCC activation, and thus the antibodies of the present application have excellent ADCC killing ability. In addition, the humanized antibodies obtained in the present application have ADCC activity comparable to that of the corresponding chimeric antibodies.

## Example 8. *In Vivo* Anti-Tumor Effects of Antibody Molecules of the Present Application

This example investigates the *in vivo* anti-tumor effects of the anti-B7H3 antibody molecules obtained in the present application in a tumor-bearing mouse model.

SPF female C.B-17-SCID mice (18-20 g) purchased from Beijing Vital River Laboratory Animal Technology Co., Ltd., with certificate No. 1100112011025061 were used in the experiment.

The A375 cells (ATCC, CRL-1619) were subcultured conventionally for the subsequent *in vivo* experiment. The A375 cells were collected by centrifugation and dispersed in PBS (1×) to prepare a cell suspension with a cell concentration of $2.5 \times 10^7$ cells/mL. On day 0, 0.2 mL of the cell suspension was subcutaneously inoculated into the right abdominal region of the C.B-17 SCID mice to establish A375 tumor-bearing mouse models.

All mice were randomized into groups with 8 mice in each group on day 0 after tumor cell inoculation. The antibody molecules were administered on days 0, 4, 7, and 11 after the inoculation, with the dosages and routes of administration and the corresponding antibodies as shown in Table 13.

Table 13.

| Group | Dosage of administration | Route of administration |
|---|---|---|
| h-IgG1 | 10mg/kg | Intraperitoneal |
| hz19A2 | 10mg/kg | Intraperitoneal |
| hz20G5 | 10mg/kg | Intraperitoneal |

Five days after inoculation, the tumor volume in each mouse was measured. The tumor volume and body weight of the mice were monitored twice a week until day 14. The relative tumor growth inhibition (TGI%) was calculated on day 14 after inoculation, and the calculation formula is as follows:

$$TGI\% = 100\% \times (\text{tumor volume of the control group} - \text{tumor volume of the treatment group})/(\text{tumor volume of the control group} - \text{tumor volume of the control group before administration}).$$

Tumor volume measurement: The maximum length of major axis (L) and maximum length of minor axis (W) of tumors were measured with a vernier caliper, and the tumor volume was calculated using the following formula: $V = L \times W^2/2$.

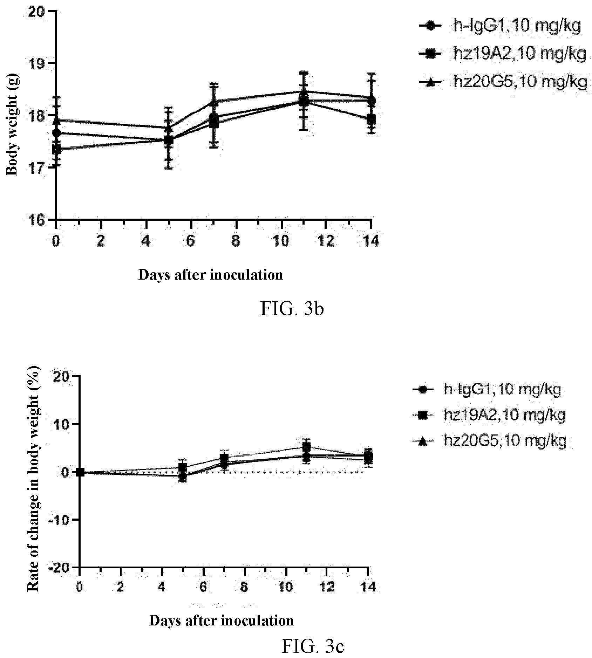

The tumor growth inhibition results are shown in FIG. 3a and Table 14: On day 14 after inoculation, the humanized antibodies hz19A2 and hz20G5 had a tumor growth inhibition of 62.4% and 46.0%, respectively, compared with the h-IgG1 control. Thus, it is shown that the humanized anti-B7H3 antibodies (hz19A2 and hz20G5) obtained in the present application have excellent anti-tumor effects.

Table 14. Tumor growth inhibition on day 14

| Group | Tumor volume (mm$^3$) | Tumor growth inhibition (%) |
|---|---|---|
| h-IgG1, 10mg/kg | 389.52 | N/A |
| hz19A2, 10mg/kg | 194.82 | 62.4 |
| hz20G5, 10mg/kg | 246.05 | 46.0 |

In addition, the body weight changes of the mice were also monitored in this experiment. As shown in FIGs. 3b-3c, there was no significant difference in the body weight of the mice in the experimental group and the control group throughout the administration period.

**Claims**

1.  An antibody or an antigen-binding fragment thereof that binds to B7-H3, comprising

    1) HCDR1, HCDR2 and HCDR3 contained in a VH set forth in SEQ ID NO: 16, and LCDR1, LCDR2 and LCDR3 contained in a VL set forth in SEQ ID NO: 17;
    2) HCDR1, HCDR2 and HCDR3 contained in a VH set forth in SEQ ID NO: 18, and LCDR1, LCDR2 and LCDR3 contained in a VL set forth in SEQ ID NO: 19;
    3) HCDR1, HCDR2 and HCDR3 contained in a VH set forth in SEQ ID NO: 20, and LCDR1, LCDR2 and LCDR3 contained in a VL set forth in SEQ ID NO: 21; or
    4) HCDR1, HCDR2 and HCDR3 contained in a VH set forth in SEQ ID NO: 22, and LCDR1, LCDR2 and LCDR3 contained in a VL set forth in SEQ ID NO: 23.

2.  An antibody or an antigen-binding fragment thereof that binds to B7-H3, comprising a heavy chain variable region VH and/or a light chain variable region VL, wherein

(I) the VH comprises HCDR1, HCDR2 and HCDR3, wherein the HCDR1 comprises or consists of an amino acid sequence set forth in any one of SEQ ID NOs: 1 and 8; the HCDR2 comprises or consists of an amino acid sequence set forth in any one of SEQ ID NOs: 2, 7, 9, and 14; the HCDR3 comprises or consists of an amino acid sequence set forth in any one of SEQ ID NOs: 3 and 10;
and/or
(ii) wherein the VL comprises LCDR1, LCDR2 and LCDR3, wherein the LCDR1 comprises or consists of an amino acid sequence set forth in any one of SEQ ID NOs: 4, 11, and 15; the LCDR2 comprises or consists of an amino acid sequence set forth in any one of SEQ ID NOs: 5 and 12; the LCDR3 comprises or consists of an amino acid sequence set forth in any one of SEQ ID NOs: 6 and 13.

3. The antibody or the antigen-binding fragment thereof according to claim 2, comprising 3 complementarity determining regions of a heavy chain variable region (HCDRs) and 3 complementarity determining regions of a light chain variable region (LCDRs), wherein

1) HCDR1 comprises or consists of an amino acid sequence set forth in SEQ ID NO: 1;

HCDR2 comprises or consists of an amino acid sequence set forth in SEQ ID NO: 2;
HCDR3 comprises or consists of an amino acid sequence set forth in SEQ ID NO: 3;
LCDR1 comprises or consists of an amino acid sequence set forth in SEQ ID NO: 4;
LCDR2 comprises or consists of an amino acid sequence set forth in SEQ ID NO: 5; and
LCDR3 comprises or consists of an amino acid sequence set forth in SEQ ID NO: 6;

2) HCDR1 comprises or consists of an amino acid sequence set forth in SEQ ID NO: 1;

HCDR2 comprises or consists of an amino acid sequence set forth in SEQ ID NO: 7;
HCDR3 comprises or consists of an amino acid sequence set forth in SEQ ID NO: 3;
LCDR1 comprises or consists of an amino acid sequence set forth in SEQ ID NO: 4;
LCDR2 comprises or consists of an amino acid sequence set forth in SEQ ID NO: 5; and
LCDR3 comprises or consists of an amino acid sequence set forth in SEQ ID NO: 6;

3) HCDR1 comprises or consists of an amino acid sequence set forth in SEQ ID NO: 8;

HCDR2 comprises or consists of an amino acid sequence set forth in SEQ ID NO: 9;
HCDR3 comprises or consists of an amino acid sequence set forth in SEQ ID NO: 10;
LCDR1 comprises or consists of an amino acid sequence set forth in SEQ ID NO: 11;
LCDR2 comprises or consists of an amino acid sequence set forth in SEQ ID NO: 12; and
LCDR3 comprises or consists of an amino acid sequence set forth in SEQ ID NO: 13; or

4) HCDR1 comprises or consists of an amino acid sequence set forth in SEQ ID NO: 8;

HCDR2 comprises or consists of an amino acid sequence set forth in SEQ ID NO: 14;
HCDR3 comprises or consists of an amino acid sequence set forth in SEQ ID NO: 10;
LCDR1 comprises or consists of an amino acid sequence set forth in SEQ ID NO: 15;
LCDR2 comprises or consists of an amino acid sequence set forth in SEQ ID NO: 12; and
LCDR3 comprises or consists of an amino acid sequence set forth in SEQ ID NO: 13.

4. The antibody or the antigen-binding fragment thereof according to any one of claims 1 to 3, comprising a heavy chain variable region VH and/or a light chain variable region VL, wherein

(a) the heavy chain variable region VH

(i) comprises or consists of an amino acid sequence having at least 90%, 91%, 92%, 93%, 94%, 95%, 96%, 97%, 98%, or 99% identity to an amino acid sequence set forth in any one of SEQ ID NOs: 16, 18, 20, and 22, and comprises the corresponding CDR sequence of the sequence; or
(ii) comprises or consists of an amino acid sequence set forth in any one of SEQ ID NOs: 16, 18, 20, and 22; or
(iii) comprises or consists of an amino acid sequence having 1 or more (preferably no more than 10, and more preferably no more than 5, 4, 3, 2, or 1) amino acid alterations (preferably amino acid replacements, and more preferably conservative amino acid replacements) compared with an amino acid sequence set forth in any one of SEQ ID NOs: 16, 18, 20, and 22, wherein preferably, the amino acid alterations do not occur in the CDRs;

and/or

(b) the light chain variable region VL

(i) comprises or consists of an amino acid sequence having at least 90%, 91%, 92%, 93%, 94%, 95%, 96%, 97%, 98%, or 99% identity to an amino acid sequence set forth in any one of SEQ ID NOs: 17, 19, 21, and 23, and comprises the corresponding CDR sequence of the sequence;

(ii) comprises or consists of an amino acid sequence set forth in any one of SEQ ID NOs: 17, 19, 21, and 23; or

(iii) comprises or consists of an amino acid sequence having 1 or more (preferably no more than 10, and more preferably no more than 5, 4, 3, 2, or 1) amino acid alterations (preferably amino acid replacements, and more preferably conservative amino acid replacements) compared with an amino acid sequence set forth in any one of SEQ ID NOs: 17, 19, 21, and 23, wherein preferably, the amino acid alterations do not occur in the CDRs.

5. The antibody or the antigen-binding fragment thereof according to claim 4, comprising

1) a heavy chain variable region VH comprising or consisting of an amino acid sequence having at least 90%, 91%, 92%, 93%, 94%, 95%, 96%, 97%, 98%, or 99% identity to an amino acid sequence set forth in SEQ ID NO: 16, and a light chain variable region VL comprising or consisting of an amino acid sequence having at least 90%, 91%, 92%, 93%, 94%, 95%, 96%, 97%, 98%, or 99% identity to an amino acid sequence set forth in SEQ ID NO: 17;

2) a heavy chain variable region VH comprising or consisting of an amino acid sequence having at least 90%, 91%, 92%, 93%, 94%, 95%, 96%, 97%, 98%, or 99% identity to an amino acid sequence set forth in SEQ ID NO: 18, and a light chain variable region VL comprising or consisting of an amino acid sequence having at least 90%, 91%, 92%, 93%, 94%, 95%, 96%, 97%, 98%, or 99% identity to an amino acid sequence set forth in SEQ ID NO: 19;

3) a heavy chain variable region VH comprising or consisting of an amino acid sequence having at least 90%, 91%, 92%, 93%, 94%, 95%, 96%, 97%, 98%, or 99% identity to an amino acid sequence set forth in SEQ ID NO: 20, and a light chain variable region VL comprising or consisting of an amino acid sequence having at least 90%, 91%, 92%, 93%, 94%, 95%, 96%, 97%, 98%, or 99% identity to an amino acid sequence set forth in SEQ ID NO: 21; or

4) a heavy chain variable region VH comprising or consisting of an amino acid sequence having at least 90%, 91%, 92%, 93%, 94%, 95%, 96%, 97%, 98%, or 99% identity to an amino acid sequence set forth in SEQ ID NO: 22, and a light chain variable region VL comprising or consisting of an amino acid sequence having at least 90%, 91%, 92%, 93%, 94%, 95%, 96%, 97%, 98%, or 99% identity to an amino acid sequence set forth in SEQ ID NO: 23.

6. The antibody or the antigen-binding fragment thereof according to any one of claims 1 to 5, comprising a heavy chain variable region and a light chain variable region selected from:

1) a heavy chain variable region VH comprising or consisting of an amino acid sequence set forth in SEQ ID NO: 16, and a light chain variable region VL comprising or consisting of an amino acid sequence set forth in SEQ ID NO: 17;

2) a heavy chain variable region VH comprising or consisting of an amino acid sequence set forth in SEQ ID NO: 18, and a light chain variable region VL comprising or consisting of an amino acid sequence set forth in SEQ ID NO: 19;

3) a heavy chain variable region VH comprising or consisting of an amino acid sequence set forth in SEQ ID NO: 20, and a light chain variable region VL comprising or consisting of an amino acid sequence set forth in SEQ ID NO: 21; or

4) a heavy chain variable region VH comprising or consisting of an amino acid sequence set forth in SEQ ID NO: 22, and a light chain variable region VL comprising or consisting of an amino acid sequence set forth in SEQ ID NO: 23.

7. The antibody or the antigen-binding fragment thereof according to any one of claims 1 to 6, comprising a heavy chain and/or a light chain, wherein

(a) the heavy chain

(i) comprises or consists of an amino acid sequence having at least 85%, 90%, 91%, 92%, 93%, 94%, 95%, 96%, 97%, 98%, or 99% identity to an amino acid sequence set forth in any one of SEQ ID NOs: 24, 26, 28, and 30, and comprising the corresponding CDR sequence of the sequence;

(ii) comprises or consists of an amino acid sequence set forth in any one of SEQ ID NOs: 24, 26, 28, and 30; or

(iii) comprises or consists of an amino acid sequence having 1 or more (preferably no more than 20 or 10, and more preferably no more than 5, 4, 3, 2, or 1) amino acid alterations (preferably amino acid replacements, and more preferably conservative amino acid replacements) compared with an amino acid sequence set forth in any one of SEQ ID NOs: 24, 26, 28, and 30, wherein preferably, the amino acid alterations do not occur in the CDRs of the heavy chain, and more preferably, the amino acid alterations do not occur in the heavy chain variable region; and/or

(b) the light chain

(i) comprises or consists of an amino acid sequence having at least 85%, 90%, 91%, 92%, 93%, 94%, 95%, 96%, 97%, 98%, or 99% identity to an amino acid sequence set forth in any one of SEQ ID NOs: 25, 27, 29, and 31, and comprising the corresponding CDR sequence of the sequence;

(ii) comprises or consists of an amino acid sequence set forth in any one of SEQ ID NOs: 25, 27, 29, and 31; or

(iii) comprises or consists of an amino acid sequence having 1 or more (preferably no more than 20 or 10, and more preferably no more than 5, 4, 3, 2, or 1) amino acid alterations (preferably amino acid replacements, and more preferably conservative amino acid replacements) compared with an amino acid sequence set forth in any one of SEQ ID NOs: 25, 27, 29, and 31, wherein preferably, the amino acid alterations do not occur in the CDRs of the light chain, and more preferably, the amino acid alterations do not occur in the light chain variable region.

8. The antibody or the antigen-binding fragment thereof according to claim 7, comprising

1) a heavy chain comprising or consisting of an amino acid sequence having at least 85%, 90%, 91%, 92%, 93%, 94%, 95%, 96%, 97%, 98%, or 99% identity to an amino acid sequence set forth in SEQ ID NO: 24, and a light chain comprising or consisting of an amino acid sequence having at least 85%, 90%, 91%, 92%, 93%, 94%, 95%, 96%, 97%, 98%, or 99% identity to an amino acid sequence set forth in SEQ ID NO: 25;

2) a heavy chain comprising or consisting of an amino acid sequence having at least 85%, 90%, 91%, 92%, 93%, 94%, 95%, 96%, 97%, 98%, or 99% identity to an amino acid sequence set forth in SEQ ID NO: 26, and a light chain comprising or consisting of an amino acid sequence having at least 85%, 90%, 91%, 92%, 93%, 94%, 95%, 96%, 97%, 98%, or 99% identity to an amino acid sequence set forth in SEQ ID NO: 27;

3) a heavy chain comprising or consisting of an amino acid sequence having at least 85%, 90%, 91%, 92%, 93%, 94%, 95%, 96%, 97%, 98%, or 99% identity to an amino acid sequence set forth in SEQ ID NO: 28, and a light chain comprising or consisting of an amino acid sequence having at least 85%, 90%, 91%, 92%, 93%, 94%, 95%, 96%, 97%, 98%, or 99% identity to an amino acid sequence set forth in SEQ ID NO: 29; or

4) a heavy chain comprising or consisting of an amino acid sequence having at least 85%, 90%, 91%, 92%, 93%, 94%, 95%, 96%, 97%, 98%, or 99% identity to an amino acid sequence set forth in SEQ ID NO: 30, and a light chain comprising or consisting of an amino acid sequence having at least 85%, 90%, 91%, 92%, 93%, 94%, 95%, 96%, 97%, 98%, or 99% identity to an amino acid sequence set forth in SEQ ID NO: 31.

9. The antibody or the antigen-binding fragment thereof according to claim 8, comprising

1) a heavy chain comprising or consisting of an amino acid sequence set forth in SEQ ID NO: 24, and a light chain comprising or consisting of an amino acid sequence set forth in SEQ ID NO: 25;

2) a heavy chain comprising or consisting of an amino acid sequence set forth in SEQ ID NO: 26, and a light chain comprising or consisting of an amino acid sequence set forth in SEQ ID NO: 27;

3) a heavy chain comprising or consisting of an amino acid sequence set forth in SEQ ID NO: 28, and a light chain comprising or consisting of an amino acid sequence set forth in SEQ ID NO: 29; or

4) a heavy chain comprising or consisting of an amino acid sequence set forth in SEQ ID NO: 30, and a light chain comprising or consisting of an amino acid sequence set forth in SEQ ID NO: 31.

10. The antibody or the antigen-binding fragment thereof according to any one of claims 1 to 9, wherein the antibody is an antibody or an antigen-binding fragment thereof in the form of IgG1, IgG2, IgG3, or IgG4, preferably an antibody in the form of IgG1, and more preferably comprises a human IgG1 Fc region.

11. The antibody or the antigen-binding fragment thereof according to any one of claims 1 to 10, wherein the antibody is a monoclonal antibody, a chimeric antibody, or a humanized antibody.

12. The antibody or the antigen-binding fragment thereof according to any one of claims 1 to 11, wherein the antigen-binding fragment is an antibody fragment selected from: Fab, Fab', Fab'-SH, Fv, a single-chain antibody such as scFv, (Fab')2 fragment, a single-domain antibody, a diabody (dAb), and a linear antibody.

13. An isolated nucleic acid, encoding the anti-B7-H3 antibody or the antigen-binding fragment thereof according to any one of the preceding claims.

14. A vector, comprising the nucleic acid according to claim 13, wherein preferably, the vector is an expression vector.

15. A host cell, comprising the nucleic acid according to claim 13 or the vector according to claim 14, wherein preferably, the host cell is prokaryotic or eukaryotic, and more preferably, an E. *coli* cell, a mammal cell (e.g., 293 cell or CHO cell), or other cells suitable for preparing an antibody or an antigen-binding fragment thereof.

16. A method for preparing an anti-B7-H3 antibody or an antigen-binding fragment thereof, comprising culturing the host cell according to claim 15 under conditions suitable for expressing a nucleic acid encoding the antibody or the antigen-binding fragment thereof according to any one of claims 1 to 12, and optionally isolating the antibody or the antigen-binding fragment thereof, wherein optionally, the method further comprises recovering the anti-B7-H3 antibody or the antigen-binding fragment thereof from the host cell.

17. An anti-B7-H3 antibody or an antigen-binding fragment thereof prepared by the method according to claim 16.

18. An immunoconjugate, comprising the antibody or the antigen-binding fragment thereof according to any one of claims 1 to 12 and 17 conjugated to a therapeutic agent or a diagnostic agent.

19. A pharmaceutical composition, comprising the antibody or the antigen-binding fragment thereof according to any one of claims 1 to 12 and 17 or the immunoconjugate according to claim 18, and optionally a pharmaceutical supplementary material.

20. Use of the antibody or the antigen-binding fragment thereof according to any one of claims 1 to 12 and 17 or the immunoconjugate according to claim 18 or the pharmaceutical composition according to claim 19 in the preparation of a medicament for the treatment and/or diagnosis of cancer or a tumor, wherein preferably, the tumor is a solid tumor.

21. A method for preventing or treating a B7-H3-related disease or disorder, such as cancer, in a subject, comprising administering to the subject an effective amount of the anti-B7-H3 antibody or the antigen-binding fragment thereof according to any one of claims 1 to 12 and 17, or the immunoconjugate according to claim 18, or the pharmaceutical composition according to claim 19.

22. A method for detecting B7-H3 in a sample, comprising

(a) contacting the sample with the anti-B7-H3 antibody or the antigen-binding fragment thereof according to any one of claims 1 to 12 and 17, or the immunoconjugate according to claim 18; and
(b) detecting a complex formed by the antibody or the antigen-binding fragment thereof or the immunoconjugate and B7-H3, wherein optionally, the antibody is detectably labeled.

**Cell-level affinity of CHOS hB7H3(4lg)**

| | hz19A2 | hz20G5 | MGA271 |
|---|---|---|---|
| EC50 | 8.505 | 6.281 | 8.536 |

**Cell-level affinity of CHOS hB7H3(2lg)**

| | hz19A2 | hz20G5 | MGA271 |
|---|---|---|---|
| EC50 | 138.5 | 7.538 | 100.3 |

FIG. 1

## Antibody-dependent cell-mediated cytotoxicity

| | hz19A2 | hz20G5 | MGA271 |
|---|---|---|---|
| EC50 | 1.920 | 0.9106 | 2.906 |

FIG. 2

FIG. 3a

FIG. 3b

FIG. 3c

## INTERNATIONAL SEARCH REPORT

| | International application No. |
|---|---|
| | **PCT/CN2021/140449** |

**A. CLASSIFICATION OF SUBJECT MATTER**

C07K 16/28(2006.01)i; A61K 39/00(2006.01)i; A61K 39/00(2006.01)i

According to International Patent Classification (IPC) or to both national classification and IPC

**B. FIELDS SEARCHED**

Minimum documentation searched (classification system followed by classification symbols)

C07K; A61K; A61P

Documentation searched other than minimum documentation to the extent that such documents are included in the fields searched

Electronic data base consulted during the international search (name of data base and, where practicable, search terms used)

CNABS, DWPI, SIPOABS, CNTXT, WOTXT, EPTXT, USTXT, JPTXT, KRTXT, CNKI, 万方数据资源系统, WANFANG DATA RESOURCE SYSTEM, PubMed, ScienceDirect, GenBank, EBI-EMBL, STN, 中国专利生物序列检索系统, Chinese Patent Biological Sequence Retrieval System: 申请人/发明人, CDR序列, 重链和轻链可变区, 重链和轻链序列检索; B7-H3, B7H3, CD276, B7RP-2, B7 homology 3 protein, 抗体, 嵌合抗体, 人源化, 免疫缀合物, 药物组合物, 癌症, antibody, chimeric antibody, humanized antibody, composition, cancer.

**C. DOCUMENTS CONSIDERED TO BE RELEVANT**

| Category* | Citation of document, with indication, where appropriate, of the relevant passages | Relevant to claim No. |
|---|---|---|
| A | CN 109963870 A (ABBVIE INC.) 02 July 2019 (2019-07-02)<br>entire document | 1-22 |
| A | WO 2020151384 A1 (SUZHOU BRIGHT SCISTAR ANTIBODY BIOTECHNOLOGY CO., LTD.) 30 July 2020 (2020-07-30)<br>entire document | 1-22 |
| A | CN 111944050 A (SUZHOU PULEKANG PHARMACEUTICAL TECHNOLOGY CO., LTD.) 17 November 2020 (2020-11-17)<br>entire document | 1-22 |
| A | CA 2834136 A1 (DAIICHI SANKYO CO., LTD.) 01 November 2012 (2012-11-01)<br>entire document | 1-22 |
| A | US 2013149236 A1 (MACROGENICS, INC.) 13 June 2013 (2013-06-13)<br>entire document | 1-22 |
| A | US 2019338030 A1 (FULL SPECTRUM GENETICS, INC.) 07 November 2019 (2019-11-07)<br>entire document | 1-22 |

☐ Further documents are listed in the continuation of Box C.    ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **08 March 2022** | **22 March 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

<table>
<tr><td>INTERNATIONAL SEARCH REPORT</td><td>International application No.<br><br>**PCT/CN2021/140449**</td></tr>
</table>

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

   a. ☑ forming part of the international application as filed:

      ☑ in the form of an Annex C/ST.25 text file.

      ☐ on paper or in the form of an image file.

   b. ☐ furnished together with the international application under PCT Rule 13ter.1(a) for the purposes of international search only in the form of an Annex C/ST.25 text file.

   c. ☐ furnished subsequent to the international filing date for the purposes of international search only:

      ☐ in the form of an Annex C/ST.25 text file (Rule 13ter.1(a)).

      ☐ on paper or in the form of an image file (Rule 13ter.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

## INTERNATIONAL SEARCH REPORT

International application No.

**PCT/CN2021/140449**

| Box No. II | Observations where certain claims were found unsearchable (Continuation of item 2 of first sheet) |

This international search report has not been established in respect of certain claims under Article 17(2)(a) for the following reasons:

1. ☑ Claims Nos.: **21**
   because they relate to subject matter not required to be searched by this Authority, namely:

   [1]   Claim 21 relates to a method for preventing or treating B7-H3 related diseases or disorders, such as cancer, in a subject, which belongs to a disease treatment method, and thus belongs to the disease treatment method defined in PCT rule 39.1(iv). The present report is made on the basis of amending claim 21 to be a claim for drug preparation.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (January 2015)

## INTERNATIONAL SEARCH REPORT
### Information on patent family members

| International application No. |
| --- |
| **PCT/CN2021/140449** |

| Patent document cited in search report | | | Publication date (day/month/year) | Patent family member(s) | | | Publication date (day/month/year) |
| --- | --- | --- | --- | --- | --- | --- | --- |
| CN | 109963870 | A | 02 July 2019 | JP | 2021006527 | A | 21 January 2021 |
| | | | | PE | 20190177 | A1 | 01 February 2019 |
| | | | | DK | 3458479 | T3 | 08 February 2021 |
| | | | | CL | 2018003520 | A1 | 15 March 2019 |
| | | | | PL | 3458479 | T4 | 26 July 2021 |
| | | | | PL | 3458479 | T3 | 26 July 2021 |
| | | | | BR | 112018075626 | A2 | 19 March 2019 |
| | | | | UA | 124198 | C2 | 04 August 2021 |
| | | | | RS | 61828 | B1 | 30 June 2021 |
| | | | | AU | 2017279550 | A1 | 03 January 2019 |
| | | | | KR | 20190015755 | A | 14 February 2019 |
| | | | | DO | P2018000276 | A | 31 December 2018 |
| | | | | SG | 10201914119 T | A | 27 February 2020 |
| | | | | MX | 2018015271 | A | 12 August 2019 |
| | | | | CR | 20180603 | A | 29 July 2019 |
| | | | | SG | 11201811193 T | A | 30 January 2019 |
| | | | | IL | 263600 | D0 | 31 January 2019 |
| | | | | CL | 2019002250 | A1 | 25 October 2019 |
| | | | | US | 2017355769 | A1 | 14 December 2017 |
| | | | | US | 10640563 | B2 | 05 May 2020 |
| | | | | WO | 2017214335 | A1 | 14 December 2017 |
| | | | | WO | 2017214335 | A4 | 08 March 2018 |
| | | | | LT | 3458479 | T | 25 February 2021 |
| | | | | PT | 3458479 | T | 01 March 2021 |
| | | | | JP | 2019528240 | A | 10 October 2019 |
| | | | | JP | 6751165 | B2 | 02 September 2020 |
| | | | | CA | 3027045 | A1 | 14 December 2017 |
| | | | | HU | E053356 | T2 | 28 June 2021 |
| | | | | PH | 12018502601 | A1 | 30 September 2019 |
| | | | | EC | SP19000282 | A | 31 January 2019 |
| | | | | UY | 37278 | A | 31 January 2018 |
| | | | | SI | 3458479 | T1 | 31 August 2021 |
| | | | | US | 2021171637 | A1 | 10 June 2021 |
| | | | | HR | P20210170 | T1 | 19 March 2021 |
| | | | | ES | 2861499 | T3 | 06 October 2021 |
| | | | | EP | 3458479 | A1 | 27 March 2019 |
| | | | | EP | 3458479 | B1 | 04 November 2020 |
| | | | | TW | 201809003 | A | 16 March 2018 |
| | | | | CO | 2018013471 | A2 | 28 December 2018 |
| | | | | EP | 3835322 | A2 | 16 June 2021 |
| | | | | EP | 3835322 | A3 | 06 October 2021 |
| | | | | RU | 2018146948 | A | 14 July 2020 |
| | | | | RU | 2018146948 | A3 | 17 June 2021 |
| | | | | RU | 2764651 | C2 | 19 January 2022 |
| WO | 2020151384 | A1 | 30 July 2020 | CN | 109851673 | A | 07 June 2019 |
| CN | 111944050 | A | 17 November 2020 | | None | | |
| CA | 2834136 | A1 | 01 November 2012 | HU | E038685 | T2 | 28 November 2018 |
| | | | | US | 2013078234 | A1 | 28 March 2013 |
| | | | | US | 9371395 | B2 | 21 June 2016 |
| | | | | CA | 2834136 | C | 17 April 2018 |

Form PCT/ISA/210 (patent family annex) (January 2015)

**INTERNATIONAL SEARCH REPORT**
Information on patent family members

| International application No. |
|---|
| **PCT/CN2021/140449** |

| Patent document cited in search report | | | Publication date (day/month/year) | Patent family member(s) | | | Publication date (day/month/year) |
|---|---|---|---|---|---|---|---|
| | | | | MX | 370696 | B | 19 December 2019 |
| | | | | ES | 2667568 | T3 | 11 May 2018 |
| | | | | JP | WO2012147713 | A1 | 28 July 2014 |
| | | | | JP | 5917498 | B2 | 18 May 2016 |
| | | | | EP | 2703486 | A1 | 05 March 2014 |
| | | | | EP | 2703486 | A4 | 25 February 2015 |
| | | | | EP | 2703486 | B1 | 07 March 2018 |
| | | | | PT | 2703486 | T | 18 May 2018 |
| | | | | CO | 6811812 | A2 | 16 December 2013 |
| | | | | ZA | 201307983 | B | 27 January 2016 |
| | | | | TW | 201249869 | A | 16 December 2012 |
| | | | | TW | I561531 | B | 11 December 2016 |
| | | | | JP | 2016165294 | A | 15 September 2016 |
| | | | | JP | 6224759 | B2 | 01 November 2017 |
| | | | | DK | 2703486 | T3 | 28 May 2018 |
| | | | | RU | 2013152164 | A | 27 May 2015 |
| | | | | RU | 2668170 | C2 | 26 September 2018 |
| | | | | KR | 20140033018 | A | 17 March 2014 |
| | | | | KR | 102030987 | B1 | 11 November 2019 |
| | | | | MX | 2013012285 | A | 21 November 2013 |
| | | | | MX | 344773 | B | 06 January 2017 |
| | | | | SG | 194620 | A1 | 30 December 2013 |
| | | | | CN | 103687945 | A | 26 March 2014 |
| | | | | IL | 229061 | D0 | 31 December 2013 |
| | | | | IL | 229061 | A | 28 February 2019 |
| | | | | SI | 2703486 | T1 | 31 May 2018 |
| | | | | US | 2016368990 | A1 | 22 December 2016 |
| | | | | TR | 201808018 | T4 | 21 June 2018 |
| | | | | PL | 2703486 | T3 | 31 July 2018 |
| | | | | HR | 2703486 | T1 | 01 June 2018 |
| | | | | RS | 57279 | B1 | 31 August 2018 |
| | | | | LT | 2703486 | T | 25 May 2018 |
| | | | | NZ | 616809 | A | 28 August 2015 |
| | | | | AU | 2012248470 | A1 | 21 November 2013 |
| | | | | AU | 2012248470 | B2 | 27 October 2016 |
| | | | | AU | 2001248470 | A1 | 01 November 2001 |
| | | | | WO | 2012147713 | A1 | 01 November 2012 |
| US | 2013149236 | A1 | 13 June 2013 | CN | 102892426 | A | 23 January 2013 |
| | | | | CL | 2018002380 | A1 | 07 December 2018 |
| | | | | CN | 106279416 | A | 04 January 2017 |
| | | | | CR | 20120450 | A | 27 December 2012 |
| | | | | GE | P20166442 | B | 10 March 2016 |
| | | | | SI | 2542256 | T1 | 29 November 2019 |
| | | | | JO | 3538 | B1 | 05 July 2020 |
| | | | | HR | P20191483 | T1 | 15 November 2019 |
| | | | | PE | 20170779 | A1 | 04 July 2017 |
| | | | | PT | 2542256 | T | 05 September 2019 |
| | | | | NZ | 701539 | A | 24 April 2015 |
| | | | | BR | 112012022210 | A2 | 05 September 2017 |
| | | | | BR | 112012022210 | B1 | 17 August 2021 |

Form PCT/ISA/210 (patent family annex) (January 2015)

**INTERNATIONAL SEARCH REPORT**
Information on patent family members

International application No.

**PCT/CN2021/140449**

| Patent document cited in search report | | | Publication date (day/month/year) | Patent family member(s) | | | Publication date (day/month/year) |
|---|---|---|---|---|---|---|---|
| | | | | NZ | 705128 | A | 24 April 2015 |
| | | | | SG | 183847 | A1 | 30 October 2012 |
| | | | | RS | 59269 | B1 | 31 October 2019 |
| | | | | TW | 201617096 | A | 16 May 2016 |
| | | | | TW | I639441 | B | 01 November 2018 |
| | | | | US | 9150656 | B2 | 06 October 2015 |
| | | | | TW | 201707724 | A | 01 March 2017 |
| | | | | TW | I645858 | B | 01 January 2019 |
| | | | | SA | 114350709 | B1 | 31 August 2015 |
| | | | | US | 2020377612 | A1 | 03 December 2020 |
| | | | | TW | 201130514 | A | 16 September 2011 |
| | | | | TW | I551296 | B | 01 October 2016 |
| | | | | MA | 34062 | B1 | 05 March 2013 |
| | | | | SG | 10201604336V | A | 28 July 2016 |
| | | | | ME | 03447 | B | 20 January 2020 |
| | | | | US | 2015274838 | A1 | 01 October 2015 |
| | | | | US | 9714295 | B2 | 25 July 2017 |
| | | | | CL | 2012002433 | A1 | 22 March 2013 |
| | | | | DK | 2542256 | T3 | 26 August 2019 |
| | | | | US | 2015259434 | A1 | 17 September 2015 |
| | | | | US | 9714296 | B2 | 25 July 2017 |
| | | | | CL | 2016000284 | A1 | 02 September 2016 |
| | | | | PL | 2542256 | T3 | 31 January 2020 |
| | | | | EP | 2982380 | A1 | 10 February 2016 |
| | | | | EP | 2982380 | B1 | 01 September 2021 |
| | | | | JP | 2013520994 | A | 10 June 2013 |
| | | | | JP | 5998060 | B2 | 28 September 2016 |
| | | | | CL | 2018002383 | A1 | 07 December 2018 |
| | | | | TW | 201439120 | A | 16 October 2014 |
| | | | | TW | I551611 | B | 01 October 2016 |
| | | | | AU | 2011223782 | A1 | 20 September 2012 |
| | | | | AU | 2011223782 | B2 | 18 September 2014 |
| | | | | GE | P201706660 | B | 25 April 2017 |
| | | | | EP | 2542256 | A2 | 09 January 2013 |
| | | | | EP | 2542256 | A4 | 12 February 2014 |
| | | | | EP | 2542256 | B1 | 22 May 2019 |
| US | 2019338030 | A1 | 07 November 2019 | US | 10865245 | B2 | 15 December 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 7332581 B
- US 6737056 B
- WO 2004056312 A
- US 6194551 B
- WO 9951642 A
- US 7371826 B
- US 5648260 A
- US 5624821 A
- WO 9429351 A
- US 7521541 B
- US 5648237 A
- US 5789199 A
- US 5840523 A
- WO 2009080251 A
- WO 2009080252 A
- WO 2009080253 A
- WO 2009080254 A
- WO 2010112193 A
- WO 2010115589 A
- WO 2010136172 A
- WO 2010145792 A
- WO 2010145793 A
- US 5208020 A
- US 20160264672 A1
- WO 11003495 A

### Non-patent literature cited in the description

- **KABAT et al.** Sequences of Proteins of Immunological Interest. Public Health Service, National Institutes of Health, 1991
- **KINDT et al.** Kuby Immunology. W. H. Freeman and Co, 2007, 91
- **PORTOLANO et al.** *J. Immunol.,* 1993, vol. 150, 880-887
- **CLARKSON et al.** *Nature,* 1991, vol. 352, 624-628
- **CHOTHIA ; LESK.** *J. mol. biol.,* 1987, vol. 196, 901-917
- **NEEDLEMA ; WUNSCH.** *J. Mol. Biol.,* 1970, vol. 48, 444-453
- **E. MEYERS ; W MILLER.** *CABIOS,* 1989, vol. 4, 11-17
- **SHIELDS et al.** *J. Biol. Chem.,* 2001, vol. 9 (2), 6591-6604
- **IDUSOGIE et al.** *J. Immunol.,* 2000, vol. 164, 4178-4184
- **DUNCAN ; WINTER.** *Nature,* 1988, vol. 322, 738-40
- **CHARLTON.** Methods in Molecular Biology. Humana Press, 2003, vol. 248, 245-254
- **GERNGROSS.** *Nat. Biotech.,* 2004, vol. 22, 1409-1414
- **LI et al.** *Nat. Biotech.,* 2006, vol. 24, 210-215
- **GRAHAM et al.** *J. Gen Virol.,* 1977, vol. 36, 59
- **URLAUB et al.** *Proc. Natl. Acad. Sci. USA,* 1980, vol. 77, 216
- **YAZAKI ; WU.** Methods in Molecular Biology. Humana Press, 2003, vol. 248, 255-268
- **CHARI et al.** *Cancer Research,* 1992, vol. 52, 127-131
- **SAMBROOK et al.** Molecular Cloning: A Laboratory Manual. 2001
- **SAMBROOK et al.** Molecular Cloning: A Laboratory Manual. 1989
- **MANIATIS et al.** Molecular Cloning: A Laboratory Manual. 1982
- **AUSUBEL et al.** Current Protocols in Molecular Biology. John Wiley and Sons, July 2008
- Short Protocols in Molecular Biology: A Compendium of Methods from Current Protocols in Molecular Biology. Greene Pub. Associates and Wiley-Interscience
- **GLOVER.** DNA Cloning: A Practical Approach. IRL Press, 1985, vol. I, II
- **ANAND.** Techniques for the Analysis of Complex Genomes. Academic Press, 1992
- Transcription and Translation. 1984
- **PERBAL.** *A Practical Guide to Molecular Cloning,* 1984
- **HARLOW ; LANE.** Antibodies. Cold Spring Harbor Laboratory Press, 1998
- Current Protocols in Immunology. 1991
- *Annual Review of Immunology, and journals and monographs such as Advances in Immunology.*
- **ESTEP, P et al.** High throughput solution Based measurement of antibody-antigen affinity and epitope binding. *mAbs,* 2013, vol. 5 (2), 270-8